# EUROPEAN PATENT APPLICATION

(11) **EP 4 190 338 A1**
(43) Date of publication of application: **07.06.2023**
(21) Application number: 21849869.9
(22) Date of filing: 26.07.2021
(51) Int. Cl.: A61K 35/28, A61P 9/00, A61P 9/10, A23L 33/10, C12N 5/0775

(54) **COMPOSITION INCLUDING STEM CELL-DERIVED EXOSOME, AND METHOD FOR PRODUCING SAME**

(30) Priority: 29.07.2020 KR 20200094588
(71) Applicant: Brexogen Inc., Seoul 05855 (KR)
(72) Inventor: KIM, Sue, Seoul 05855 (KR); JEONG, Seon Yeong, Yongin-si, Gyeonggi-do 17145 (KR)
(74) Representative: Plougmann Vingtoft a/s
(86) International application number: PCT/KR2021/009630
(87) International publication number: WO 2022/025559

(57) **Abstract**

The present invention relates to a composition including a stem cell-derived exosome, and a method for producing same. A composition according to the present invention has excellent effects in terms of anti-inflammatory effects, fibrosis inhibition, vascular endothelial cell proliferation, blood vessel formation, survival rate improvement, and protective regeneration of cardiomyocytes, and thus can be used as an agent for preventing or treating heart disease, inflammatory disease, immune disease, fibrotic disease, or vascular disease.

## Description

### Technical Field

The present disclosure relates to a composition containing stem cell-derived exosomes and preparation method therefor and, more specifically, to a composition containing exosomes derived from mesenchymal stem cells or a culture thereof, which are superb in terms of anti-inflammation, anti-fibrotic activity, vascular endothelial proliferation, vascularization, viability improvement, and myocardial protection and regeneration effects.

### Background Art

Extracellular vesicles are vesicles, composed of a lipid bilayer, which are spherical with a size of 30-1,000 nm, as exemplified by microvesicles, exosomes, and the like.

Exosomes have lipid bilayers that have the same phospholipid bilayer structure as in the source cells thereof (donor cells), and are compositions of the substances extracellularly released by the cells, playing functional roles such as cell-cell communication and cellular immune intervention.

Exosomes carry cell-specific constituents accounting for biological functions characteristic of cells of origin and include various water-soluble proteins, peripheral proteins, and transmembrane proteins in addition to phospholipids, mRNA, and miRNA.

Such exosomes are released from all animal cells such as mast cells, lymphocytes, astrocytes, platelets, nerve cells, endothelial cells, epithelial cells, etc. and are found in various body fluids including blood, urine, mucus, saliva, bile juice, ascitic fluid, cerebrospinal fluid, and so on. Exhibiting highly selective penetration sufficient to cross even the blood-brain barrier (BBB) as well as cell membranes of epidermal and endothelial cells, exosomes can find applications in the development of drug delivery systems (DDS) utilizing nanocarriers for specific drugs.

Exosomes and microvesicles released from mesenchymal stem cells are involved in cell-to-cell communication and show medicinally regenerative therapeutic efficacy that stem cells possess.

Exosomes are known to bring about a trophic effect into paracrine factors secreted from stem cells that have been transplanted in vivo without survival for a long period of time. Released into such factors are small molecules such as growth factors, chemokines, cytokines, etc. by extracellular vesicles such as exosomes, and such exosomes are derived from stem cells. Therefore, exosomes are utilized for characterizing stem cells and evaluating therapeutic efficacies thereof. In recent years, active research into therapeutic effects of exosomes secreted by mesenchymal stem cells, but not mesenchymal stem cells themselves, on various diseases have been ongoing. In the academia and in various industrial fields, it is expected that this strategy might be a new promising alternative that can surmount the limitation of conventional stem cell therapies.

### Disclosure of Invention

### Technical Problem

The present inventors succeeded in developing a composition isolated from stem cells or a culture thereof and found that the composition according to the present disclosure is highly superb in terms of anti-inflammatory activity, fibrosis inhibition, vascular endothelial cell proliferation, blood vessel formation, viability improvement, and protective regeneration of cardiomyocytes.

Accordingly, an aspect of the present disclosure is to provide a composition including stem cell-derived exosomes.

Another aspect of the present disclosure is to provide a method for preparing a composition including stem cell-derived exosomes.

Another aspect of the present disclosure is to provide a composition including stem cell-derived exosomes for alleviation, suppression, prevention, or treatment of a heart disease.

Another aspect of the present disclosure is to provide a method for preparing a composition for alleviation, suppression, prevention, or treatment of a heart disease.

Another aspect of the present disclosure is to provide a method for alleviating or treating a heart disease.

Another aspect of the present disclosure is to provide a use of a stem cell-derived composition for alleviating, suppressing, preventing, or treating a heart disease.

Another aspect of the present disclosure is to provide a composition including stem cell-derived exosomes for alleviation, suppression, prevention, or treatment of an inflammatory disease.

Another aspect of the present disclosure is to provide a method for preparing a composition for alleviation, suppression, prevention, or treatment of an inflammatory disease.

Another aspect of the present disclosure is to provide a method for alleviating or treating an inflammatory disease.

Another aspect of the present disclosure is to provide a use of a stem cell-derived composition for alleviating, suppressing, preventing, or treating an inflammatory disease.

Another aspect of the present disclosure is to provide a composition including stem cell-derived exosomes for alleviation, suppression, prevention, or treatment of an immune disease.

Another aspect of the present disclosure is to provide a method for preparing a composition for alleviation, suppression, prevention, or treatment of an immune disease.

Another aspect of the present disclosure is to provide a method for alleviating or treating an immune disease.

Another aspect of the present disclosure is to provide a use of a composition including stem cell-derived exosomes for alleviating, suppressing, preventing, or treating an immune disease.

Another aspect of the present disclosure is to provide a composition including stem cell-derived exosomes for alleviation, suppression, prevention, or treatment of a fibrotic disease.

Another aspect of the present disclosure is to provide a method for preparing a composition for alleviation, suppression, prevention, or treatment of a fibrotic disease.

Another aspect of the present disclosure is to provide a method for alleviating or treating a fibrotic disease.

Another aspect of the present disclosure is to provide a use of a stem cell-derived composition for alleviating, suppressing, preventing, or treating a fibrotic disease.

Another aspect of the present disclosure is to provide a composition including stem cell-derived exosomes for alleviation, suppression, prevention, or treatment of a vascular disease.

Another aspect of the present disclosure is to provide a method for preparing a composition for alleviation, suppression, prevention, or treatment of a vascular disease.

Another aspect of the present disclosure is to provide a method for alleviating or treating a vascular disease.

Another aspect of the present disclosure is to provide a use of a stem cell-derived composition for alleviating, suppressing, preventing, or treating a vascular disease.

### Solution to Problem

The present disclosure relates to a composition including stem cell-derived exosomes and a preparation method therefor wherein the composition exhibits excellent therapeutic or prophylactic effects on heart diseases, inflammatory diseases, immune diseases, fibrotic diseases, skin detects, and vascular diseases.

It was found in the present disclosure that the composition according to the present disclosure has an anti-inflammatory activity and an inhibitory activity against fibrosis and can enhance the proliferation, formation, or viability of vascular endothelial cells and protect and regenerate cardiomyocytes.

Below, a detailed description will be given of the present disclosure.

An aspect of the present disclosure is drawn to a composition including stem cell-derived exosomes.

As used herein, the term "exosome" refers to a cell-derived vesicle. Exosomes are found in body fluids of almost all eukaryotic organisms. Exosomes are about 30-100 nm in size, larger than LDL proteins, but far smaller than erythrocytes. When multivesicular bodies fuse to cell membranes, exosomes are secreted from cells or just from the cell membranes. Such exosomes are well known to perform important and specialized functions such as coagulation, cell-to-cell signaling, etc.

As used herein, the term "stem cell" refers to an undifferentiated cell that can self-renew and differentiate into two or more different types of cells.

In an embodiment of the present disclosure, the stem cells may be autogenous or homogenous stem cells, may be originated from any type of animals including humans and non-human mammals, and may be derived from adults or embryos. In detail, the stem cells may be selected from the group consisting of embryonic stem cells, adult stem cells, induced pluripotent stem cells (iPSC), iPSC-derived mesenchymal stem cells, BxC stem cells, BxC-A1 stem cells, BxC-I10 stem cells, BxC-G63 stem cells, BxC-R11 stem cells, and BxC-R56 stem cells, but with no limitations thereto.

As used herein, the term "adult stem cell" refers to an undifferentiated cell just before differentiation to cells of specific organs, found in umbilical cord blood, adult bone marrow, blood, etc., which has the ability to develop to tissues of the body as needed.

In an embodiment of the present disclosure, the adult stem cells may be selected from the group consisting of adult stem cells from tissues of human or animal origin, mesenchymal stromal cells from tissues of human or animal origin, and mesenchymal stromal cells and multipotent stem cells derived from induced pluripotent stem cells from tissues of human or animal origin, but with no limitations thereto.

In the present disclosure, the tissue of human or animal origin may be selected from the group consisting of umbilical cord, umbilical cord blood, lipid, muscle, nerve, skin, amnion, and placenta, but with no limitation thereto.

The stem cells from various tissues of human or animal origin may be selected from the group consisting of hematopoietic stem cells, mammary stem cells, intestinal stem cells, vascular endothelial progenitor cells, neural stem cells, olfactory neural stem cell, and testicular stem cells, but with no limitations thereto.

As used herein, the term "embryonic stem cell" refers to a cell isolated during the embryogenesis, which is derived from the inner cell mass of a blastocyst formed prior to implantation of the fertilized egg in the uterus, and cultured in vitro.

Embryonic stem cells are cells that are pluripotent or totipotent and give rise to differentiation to cells of all tissues and exhibit self-renewal, and encompass embryoid bodies derived therefrom in a wide sense.

The embryonic stem cells may be derived from any source such as humans, monkeys, pigs, horses, cattle, sheep, dogs, cats, mice, rabbits, etc., but with no limitations thereto.

As used herein, the term "induced pluripotent stem cell" (iPSC) means pluripotent cells derived from differentiated cells through artificial dedifferentiation and may be interchangeably used with "dedifferentiated stem cell".

The artificial dedifferentiation may be conducted by introducing dedifferentiation factors through viral mediation using retroviruses, lentiviruses, and sendai viruses, through non-viral mediation with the aid of non-viral vectors, proteins, and cell extracts, or through stem cell extracts, compounds, etc.

Induced pluripotent stem cells exhibit almost the same characteristics as embryonic stem cells. In detail, they are common in the aspects including cellular morphological similarity, similar gene and protein expression pattern, totipotency in vitro and in vivo, teratoma formation, creation of chimeric mice upon insertion into murine blastocysts, and germline transmission of genes.

In an embodiment of the present disclosure, the stem cells may be BxC stem cells.

In an embodiment of the present disclosure, the composition may contain BxC stem cell-derived exosomes.

The term "mesenchymal stem cells" (MSC), as used herein, refers to stem cells derived from the mesenchyme. The mesenchymal stem cells may differentiate into at least one selected from the group consisting of osteoblasts, chondrocytes, adipocytes, and myocytes. The mesenchymal stem cells can be isolated from any type of adult tissues, for examples, from bone marrow, adipose tissues, umbilical cord, or peripheral blood. MSC populations are defined by their characteristic phenotypes. MSC populations differentiated from iPSC may exhibit the same phenotype as in typical MSC populations, which may be understood to express CD105, CD73, and CD90 markers at a level of 95% or higher and CD45, CD34, and SSEA-4 at a level of 2% or less.

As used herein, the term "BxC stem cells" refers to cells that can be prepared by culturing induced pluripotent stem cells, isolating a population of the induced pluripotent stem cells (iPSC) which do not express stage-specific embryonic antigen 4 (SSEA-4) protein, and then further culturing the population of stem cells. The BxC stem cells are cells in the stage just before complete differentiation from induced pluripotent stem cells into mesenchymal stem cells, and can have the properties of complete mesenchymal stem cells through additional culturing. Therefore, the BxC stem cell population does not exhibit completely identical phenotype to that of the mesenchymal stem cell population, and may be similar in phenotype to the mesenchymal stem cell population in the range of 96% to 99.9%. For instance, CD90 protein may be expressed at a level of 0.3 % in the iPSC population, at a level of 99.7% in the mesenchymal stem cell population, and at a level of 96.9% in the BxC stem cell population, which amounts to about 98% of the expression level in the mesenchymal stem cells. Accordingly, BxC stem cells can be defined as stem cells into which the induced pluripotent stem cells, obtained as stem cells expressing no SSEA-4 proteins upon culturing induced pluripotent stem cells, are not completely differentiated into mesenchymal stem cells, but at a level of 96% to 99.9%. The BxC stem cells are superior in terms of stemness to mesenchymal stem cells differentiated from the same induced pluripotent stem cells, and can secrete a large amount of functionality-related proteins. In detail, the BxC stem cells of the present disclosure are 10 or more times greater in proliferative capacity than mesenchymal stem cells (MSCs) derived from the same tissue after undergoing nine passages and are not observed to decrease in proliferative capacity even after 12 or more passages. In addition, the expression level of Ki67, a marker related to cell proliferative capacity, is more than twice higher in BxC stem cells than in general mesenchymal stem cells. Compared to mesenchymal stem cells differentiated the same induced pluripotent stem cells, the BxC stem cells can express a higher level of at least one gene selected from the group consisting of ANKRD1, CPE, NKAIN4, LCP1, CCDC3, MAMDC2, CLSTN2, SFTA1P, EPB41L3, PDE1C, EMILIN2, SULT1C4, TRIM58, DENND2A, CADM4, AIF1L, NTM, SHISA2, RASSF4, and ACKR3 and a lower level of at least one gene selected from the group consisting of DHRS3, BMPER, IFI6, PRSS12, RDH10, and KCNE4.

The term "sternness", as used herein, is intended to encompass the capability for pluripotency of differentiating into all types of cells and for self-renewal of dividing indefinitely to produce more of the same stem cell. In detail, stemness accounts for at least one of the capabilities for increasing proliferation of stem cells while maintaining stem cells in an undifferentiated state, for increasing telomerase activity, for increasing expression of stemness acting signals, and for increasing cell mobility.

In an embodiment of the present disclosure, the stem cells may be pretreated with at least one selected from the group consisting of pioglitazone, phorbol 12-myristate 13-acetate (PMA), exendin-4, hyaluronic acid, and resveratrol.

As used herein, the term "pretreatment" refers to a process of culturing mesenchymal stem cells in a medium containing a specific material. For example, the pretreatment means a process in which mesenchymal stem cells completely differentiated from iPSCs are further cultured in a medium containing at least one selected from the group consisting of pioglitazone, phorbol 12-myristate 13-acetate (PMA), exendin-4, hyaluronic acid, and resveratrol.

In an embodiment of the present disclosure, the induced pluripotent stem cell-derived mesenchymal stem cells may result from pretreatment with at least one selected from the group consisting of pioglitazone, phorbol 12-myristate 13-acetate, exendin-4, hyaluronic acid, and resveratrol.

In an embodiment of the present disclosure, the induced pluripotent stem cell-derived mesenchymal stem cells may result from pretreatment with hyaluronic acid.

In an embodiment of the present disclosure, the induced pluripotent stem cell-derived mesenchymal stem cells may result from pretreatment with pioglitazone.

In an embodiment of the present disclosure, the induced pluripotent stem cell-derived mesenchymal stem cells may result from pretreatment with phorbol 12-myristate 13-acetate.

In an embodiment of the present disclosure, the induced pluripotent stem cell-derived mesenchymal stem cells may result from pretreatment with exendin-4.

In an embodiment of the present disclosure, the induced pluripotent stem cell-derived mesenchymal stem cells may result from pretreatment with resveratrol.

In the present disclosure, pioglitazone, phorbol 12-myristate 13-acetate, exendin-4, hyaluronic acid, and resveratrol can enhance stemness and proliferative capacity of stem cells and increase the number of stem cell-derived exosomes and the contents of proteins and RNA in the exosomes.

In the present disclosure, the composition may contain at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11e), and BxC-R56 stem cell-derived exosomes (BxC-R56e) .

In an embodiment of the present disclosure, the composition may contain BxC-A1 stem cell-derived exosomes.

As used herein, the term "BxC-A1 stem cells" refers to mesenchymal stem cells obtained by culturing BxC stem cells according to the present disclosure to conduct complete differentiation into mesenchymal stem cells and then culturing the same in a medium containing pioglitazone. For example, the BxC-A1 stem cells may be obtained by culturing BxC stem cells to preform complete differentiation into mesenchymal stem cells and then culturing (pretreating) the same in a medium containing pioglitazone at a concentration of 0.001 to 1000 µM, for example, 3 µM, for 12 to 48 hours.

In an embodiment of the present disclosure, the composition may contain exosomes derived from BxC-I10 stem cells.

As used herein, the term "BxC-I10 stem cells" refers to mesenchymal stem cells obtained by culturing BxC stem cells according to the present disclosure to perform complete differentiation into mesenchymal stem cells and then culturing the same in a medium containing phorbol 12-myristate 13-acetate (PMA). For example, the BxC-I10 stem cells may be obtained by culturing BxC stem cells to preform complete differentiation into mesenchymal stem cells and then culturing (pretreating) the same in a medium containing phorbol 12-myristate 13-acetate at a concentration of 1 to 1000 µM, for example, 50 nM, for 12 to 48 hours. The BxC-I10 stem cells increase in proliferative capacity by about 220% and in exosome productivity and exosome-derived protein level by more than five times, compared to the mesenchymal stem cells treated with no substances.

In an embodiment of the present disclosure, the composition may contain BxC-G63 stem cell-derived exosomes.

As used herein, the term "BxC-G63 stem cells" refer to mesenchymal stem cells mesenchymal stem cells obtained by culturing BxC stem cells according to the present disclosure to perform complete differentiation into mesenchymal stem cells and then culturing (pretreating) the same in a medium containing exendin-4. For example, the BxC-G63 stem cells may be obtained by culturing BxC stem cells to preform complete differentiation into mesenchymal stem cells and then culturing the same in a medium containing exending-4 at a concentration of 0.01 to 1000 µM, for example, 20 nM, for 12 to 48 hours. The BxC-G63 stem cells increase in proliferative capacity by about 340%, in exosome productivity by about six times, and in exosome-derived protein level by more than five times, compared to the mesenchymal stem cells treated with no substances.

In an embodiment of the present disclosure, the composition may contain BxC-R11 stem cell-derived exosomes.

As used herein, the term "BxC-R11 stem cells" refer to mesenchymal stem cells obtained by culturing BxC stem cells according to the present disclosure to perform complete differentiation into mesenchymal stem cells and then culturing (pretreating) the same in a medium containing hyaluronic acid. For example, the BxC-R11 stem cells may be obtained by culturing BxC stem cells to perform complete differentiation into mesenchymal stem cells and then culturing the same in a medium containing hyaluronic acid at a concentration of 0.01 to 1000 ug/ml, for example, 40 µg/ml, for 12 to 48 hours. The BxC-R11 stem cells increase in proliferative capacity by about 360%, in exosome productivity by about five times, and in exosome-derived protein level by more than five times, compared to the mesenchymal stem cells treated with no substances.

In an embodiment of the present disclosure, the composition may contain BxC-R56 stem cell-derived exosomes.

As used herein, the term "BxC-R56 stem cells" refer to mesenchymal stem cells mesenchymal stem cells obtained by culturing BxC stem cells according to the present disclosure to perform complete differentiation into mesenchymal stem cells and then culturing (pretreating) the same in a medium containing resveratrol. For example, the BxC-R56 stem cells may be obtained by culturing BxC stem cells to preform complete differentiation into mesenchymal stem cells and then culturing the same in a medium containing resveratrol at a concentration of 0.01 to 10000 nM, for example, 10 nM, for 12 to 48 hours. The BxC-R56 stem cells increase in proliferative capacity by about 1,100%, in exosome productivity by about five times, and in exosome-derived protein level by more than five times, compared to the mesenchymal stem cells treated with no substances.

In an embodiment of the present disclosure, the pretreatment of the cells may be carried out with pioglitazone at a concentration of 0.001 to 1000 µM, 0.005 to 500 µM, 0.01 to 100 µM, 0.05 to 50 µM, 0.1 to 25 µM, 0.5 to 15 µM, 1 to 10 µM, or 2 to 6 µM, for example, at a concentration of 3 µM, but with no limitations thereto.

In an embodiment of the present disclosure, the pretreatment of the cells may be carried out with phorbol 12-myristate 13-acetateat a concentration of 1 to 1000 nM, 5 to 500 nM, 10 to 250 nM, 10 to 100 nM, 15 to 80 nM, or 20 to 70 nM, for example, at a concentration of 50 nM, but with no limitations thereto.

In an embodiment of the present disclosure, the pretreatment of the cells may be carried out with exendin-4 at a concentration of 0.01 to 10000 nM, 0.1 to 5000 nM, 1 to 1000 nM, 1 to 100 nM, 10 to 100 nM, 10 to 50 nM, or 10 to 30 nM, for example, at a concentration of 20 nM, but with no limitations thereto.

In an embodiment of the present disclosure, the pretreatment of the cells may be carried out with hyaluronic acid at a concentration of 0.1 to 1000 µg/ml, 0.5 to 1000 µg/ml, 1 to 500 µg/ml, 1 to 200 µg/ml, 1 to 100 µg/ml, 1 to 80 µg/ml, 1 to 60 µg/ml, or 10 to 60 ug/ml, for example, at a concentration of 40 µg/ml, but with no limitations thereto.

In an embodiment of the present disclosure, the pretreatment of the cells may be carried out with resveratrol at a concentration of 0.01 to 10000 nM, 0.1 to 5000 nM, 0.5 to 1000 nM, 1 to 500 nM, 1 to 100 nM, 5 to 100 nM, or 5 to 30 nM, for example, at a concentration of 10 nM, but with no limitations thereto.

Another aspect of the present disclosure is drawn to a pharmaceutical composition including stem cell-derived exosomes as an active ingredient.

In an embodiment of the present disclosure, the pharmaceutical composition may include exosomes derived from induced pluripotent stem cell-derived mesenchymal stem cells or a culture thereof as an active ingredient.

In an embodiment of the present disclosure, the pharmaceutical composition may include exosomes derived from stem cells pretreated with at least one selected from the group consisting of pioglitazone, phorbol 12-myristate 13-acetate (PMA), exendin-4, hyaluronic acid, and resveratrol.

In the present disclosure, the pharmaceutical composition may include exosomes derived from induced pluripotent stem cell-derived mesenchymal stem cells or a culture thereof pretreated with at least one selected from the group consisting of pioglitazone, phorbol 12-myristate 13-acetate (PMA), exendin-4, hyaluronic acid, and resveratrol.

In the present disclosure, the pharmaceutical composition may include at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11), and BxC-R56 stem cell-derived exosomes (BxC-R56e).

The term "including as an active ingredient", as used herein, means including exosomes separated from stem cells or a culture thereof at a sufficient amount to achieve alleviative, suppressive, prophylactic, or therapeutic activity for certain diseases.

In the present disclosure, the pharmaceutical composition may contain a pharmaceutically acceptable carrier, for example, lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but with no limitations thereto.

In the present disclosure, the pharmaceutical composition may further include a lubricant, a humectant, a sweetener, a flavoring agent, an emulsifier, a suspending agent, a preservative, and the like.

In the present disclosure, the pharmaceutical composition may be administered orally or parenterally, for example, via intravenous, subcutaneous, intramuscular, intraperitoneal, topical, intranasal, intrapulmonary, rectal, intrathecal, intraocular, percutaneous, and transdermal routes., but with no limitations thereto.

In the present disclosure, the dose of the pharmaceutical composition may vary depending on various factors including the method of formulation, the manner of administration, patient's age, body weight, sex, disease state, food, time of administration, route of administration, excretion rate, and response sensitivity. A skilled physician can easily determine and prescribe a dose effective for desired therapy or prophylaxis. According to an embodiment of the present disclosure, a daily dose of the pharmaceutical composition of the present disclosure is within a range of 0.0001-1000 mg/kg.

The pharmaceutical composition of the present disclosure may be formulated using a pharmaceutically acceptable carrier and/or excipient according to a method that could be easily performed by a person having ordinary skills in the art to which the present disclosure pertains, and the composition of the present disclosure may be prepared into a unit dosage form or may be contained in a multi-dose container. Here, the formulation may be in the form of a solution in an oily or aqueous medium, a suspension, an emulsion, an extract, a pulvis, a suppository, a powder, granules, a tablet, or a capsule, and may further include a dispersant or a stabilizer, but with no limitations thereto.

The dose of the pharmaceutical composition according to the present disclosure may be determined in the light of the relevant circumstances, including the patient's age, weight, and sex, the mode of administration, the state of health, the severity of the patient's symptoms, and the like. According to the judgement of physicians or pharmacists, the composition may be administered once to several times as divided a day at certain intervals. For example, the daily dose may be 1 to 1000 µg/ml on the basis of content of the active ingredient. These amounts are illustratively given for average cases and may differ from one individual to another.

In the present disclosure, the pharmaceutical composition may be for use in alleviating, suppressing, preventing, or treating a heart disease.

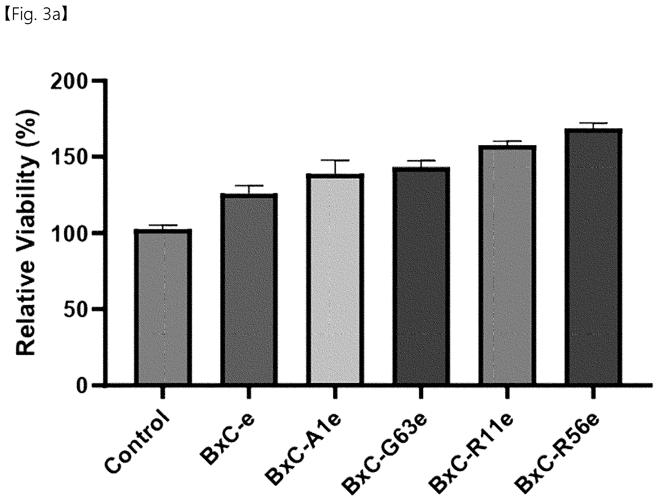

The pharmaceutical composition according to the present disclosure exhibits excellent alleviative, suppressive, prophylactic, or therapeutic effects on a heart disease. Particularly, at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11), and BxC-R56 stem cell-derived exosomes (BxC-R56e) improves proliferative rates and viability in vascular endothelial cells and have excellent regenerative and protective effects on cardiomyocytes, compared to exosomes isolated from stem cells that have not been any of the substances, thus finding applications in alleviating, suppressing, preventing, and treating a heart disease (see FIGS. 3a to 6c and 13a to 13d).

In the present disclosure, the "heart disease" may be selected from the group consisting of angina pectoris, myocardial infarction, valvular disease, cardiac failure, cardiac hypertrophy, arrhythmia, pericarditis, and endocarditis, but is not limited thereto.

In an embodiment of the present disclosure, the heart disease may be an ischemic heart disease.

As used herein, the term "ischemic heart disease" refers to the narrowing of the coronary arteries supplying blood to the heart, resulting in ischemia due to insufficient blood supply to the heart muscle. Angina pectoris and myocardial infarction are representative of heart diseases. In a severe case of heart disease, cardiac arrest may occur. Chest pain is a typical symptom, but in advanced cases, the heart function decreases to the heart failure, giving rise to breathing difficulty and arrhythmias. Arteriosclerosis has been pointed out as a main cause.

In an embodiment of the present disclosure, the ischemic heart disease may be selected from the group consisting of angina pectoris, myocardial infarction, and heart failure, but with no limitations thereto.

In the present disclosure, the pharmaceutical composition may be for use in alleviating, suppressing, preventing, or treating an inflammatory disease.

In the present disclosure, the "inflammatory disease" may be at least one selected from the group consisting of atopic dermatitis, edema, dermatitis, allergy, asthma, conjunctivitis, periodontitis, rhinitis, otitis media, pharyngitis, tonsillitis, pneumonia, gastric ulcer, gastritis, Crohn's disease, colitis, hemorrhoids, gout, ankylosing spondylitis, rheumatic lupus, fibromyalgia, psoriatic arthritis, osteoarthritis, rheumatoid arthritis, periarthritis, tendinitis, tendinitis, myositis, hepatitis, cystitis, nephritis, Sjogren's syndrome, and multiple sclerosis , but is not limited thereto.

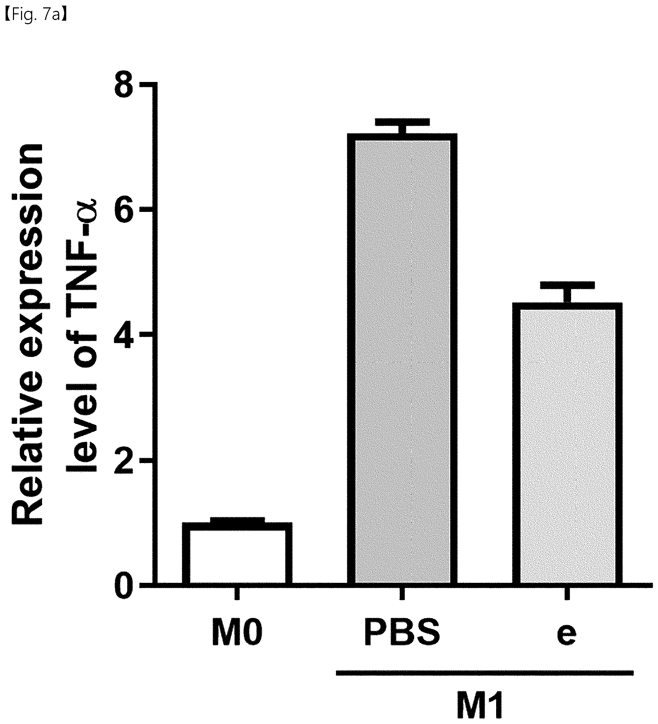

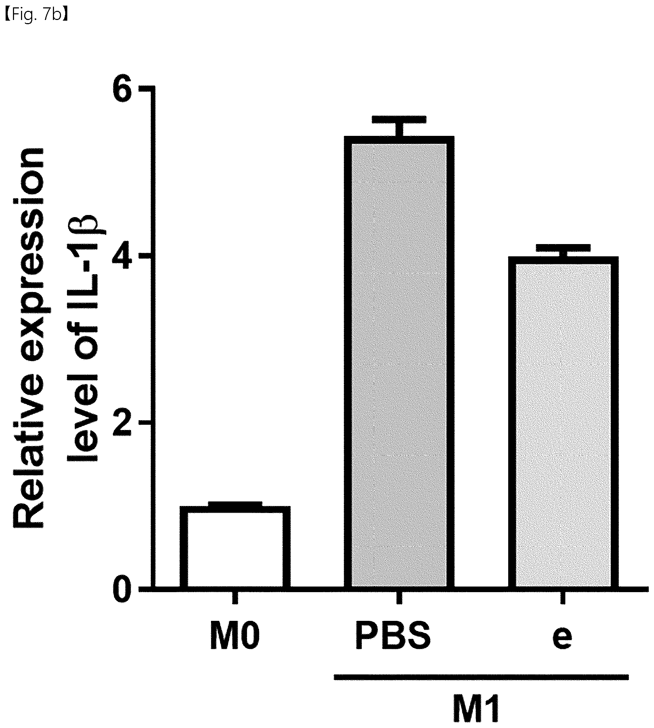

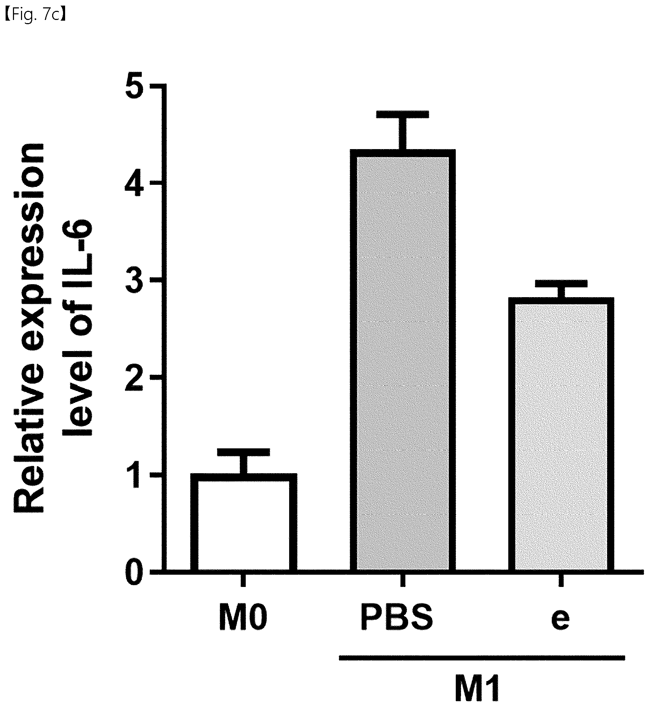

The pharmaceutical composition according to the present disclosure has excellent palliative, suppressive, prophylactic, or therapeutic effects on an inflammatory disease. In an exemplary embodiment of the present disclosure, macrophages in which inflammatory responses had been provoked by LPS stimulation were observed to greatly decrease in expression levels of TNF-α, IL-1β, and IL-6 when treated with the pharmaceutical composition according to the present disclosure, compared to the control (see FIGS. 7a to 8c) .

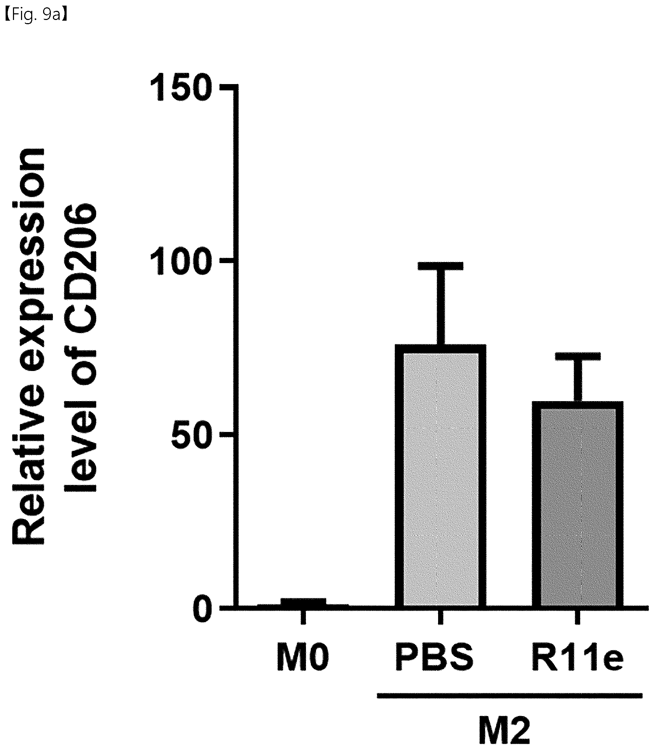

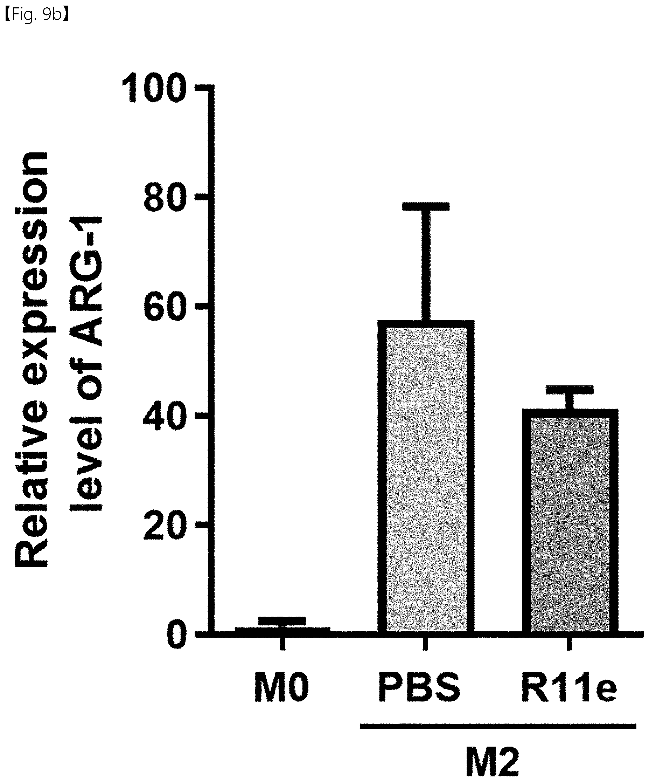

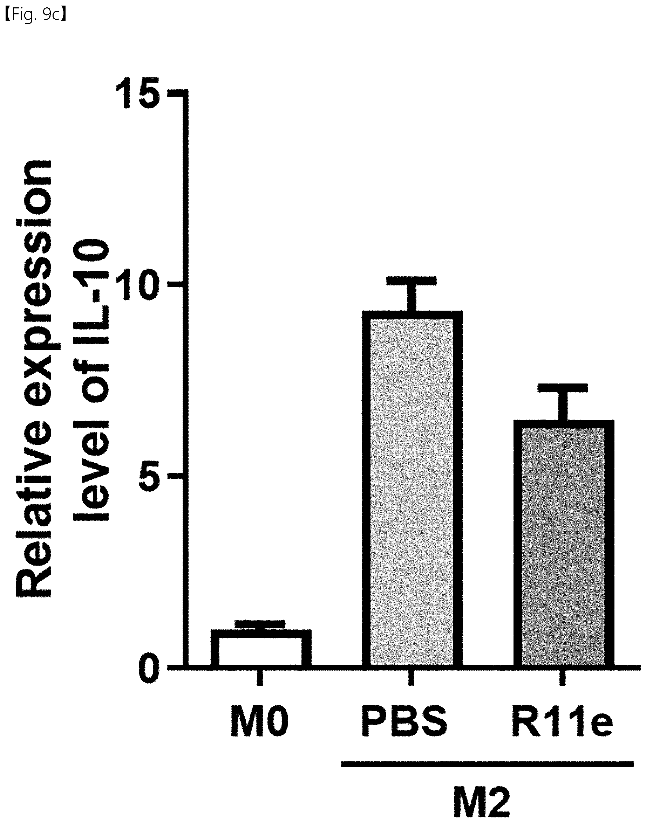

In some particular embodiments of the present disclosure, it was observed that when treated with the pharmaceutical composition according to the present disclosure, the regenerative macrophages induced by IL-4 and IL-13 were measured to not greatly decrease in expression levels of CD206, ARG-1, and IL-10, compared to the control (FIGS. 9a to 9c).

In the present disclosure, the pharmaceutical composition may be for use in alleviating, suppressing, preventing, or treating an immune disease.

As used herein, the term "immune disease" refers to a condition arising from an abnormal immune response. The immune disease may be an autoimmune disease, transplant rejection, or graft-versus-host disease.

Examples of the autoimmune disease include Crohn's disease, erythematosus, atopy, rheumatoid arthritis, Hashimoto's thyroiditis, pernicious anemia, Addison's disease, type 1 diabetes, lupus, chronic fatigue syndrome, fibromyalgia, hypothyroidism and hyperthyroidism, scleroderma, Behcet's disease, inflammatory bowel disease, multiple sclerosis, myasthenia gravis, Meniere's syndrome, Guilian-Barre syndrome, Sjogren's syndrome, vitiligo, endometriosis, psoriasis, vitiligo, systemic scleroderma, asthma, and ulcer, but are not limited thereto.

The pharmaceutical composition according to the present disclosure has excellent palliative, suppressive, prophylactic, or therapeutic effects on an immune disease. In an exemplary embodiment of the present disclosure, macrophages in which inflammatory responses had been provoked by LPS stimulation were observed to greatly decrease in expression levels of TNF-α, IL-1β, and IL-6 when treated with the pharmaceutical composition according to the present disclosure, compared to the control (see FIGS. 7a to 8c) .

In some particular embodiments of the present disclosure, it was observed that when treated with the pharmaceutical composition according to the present disclosure, the regenerative macrophages induced by IL-4 and IL-13 were measured to not greatly decrease in expression levels of CD206, ARG-1, and IL-10, compared to the control (FIGS. 9a to 9c).

In the present disclosure, the pharmaceutical composition may be for use in alleviating, suppressing, preventing, or treating a fibrotic disease.

As used herein, the term "fibrotic disease" refers to a disease in which a part of an organ hardens due to various causes, as exemplified by renal fibrosis, hepatic fibrosis, pulmonary fibrosis, and the like, with no limitations thereto.

As used herein, the term "renal fibrosis" refers to a symptom in which kidney tissues or blood vessels harden and lose renal functions due to various causes, such as an excessive inflammatory reaction occurring in the kidney tissue, fibrosis of epithelial cells, and the like.

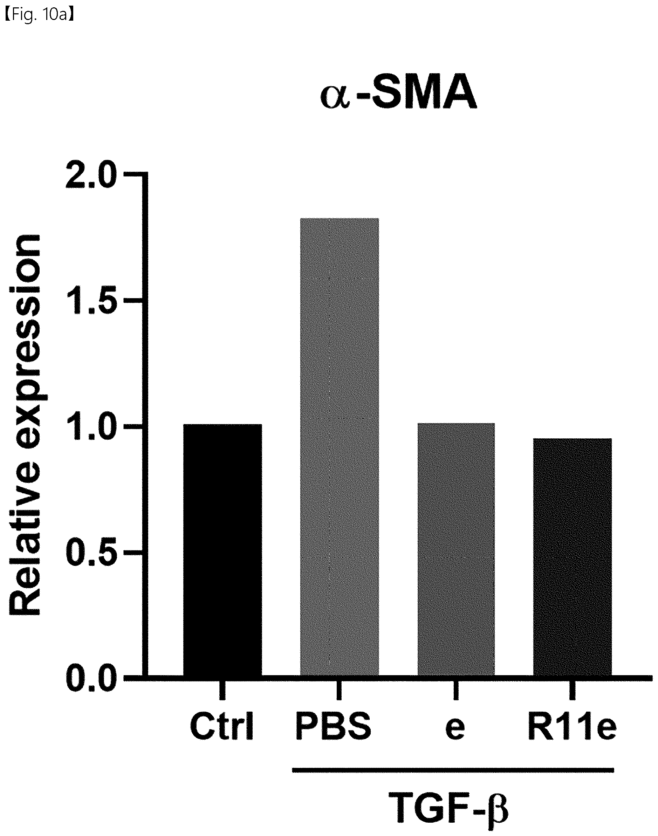

The pharmaceutical composition according to the present disclosure has excellent palliative, suppressive, prophylactic, or therapeutic effects on a fibrotic disease. In an exemplary embodiment of the present disclosure, fibroblasts in fibrosis had been induced by TGF-β1 were observed to greatly decrease in expression levels of α-SMA and CTGF when treated with the pharmaceutical composition according to the present disclosure, compared to the control (see FIGS. 10a to 12b) .

In the present disclosure, the pharmaceutical composition may be for use in alleviating, suppressing, preventing, or treating a vascular disease.

As used herein, the term "vascular disease" refers to a disease in which the intima of blood vessels is thickened by damage to vascular tissue. Examples of the vascular disease include vascular hypertrophy, unstable angina, acute myocardial infarction, atherosclerosis, and in-stent restenosis, but are not limited thereto.

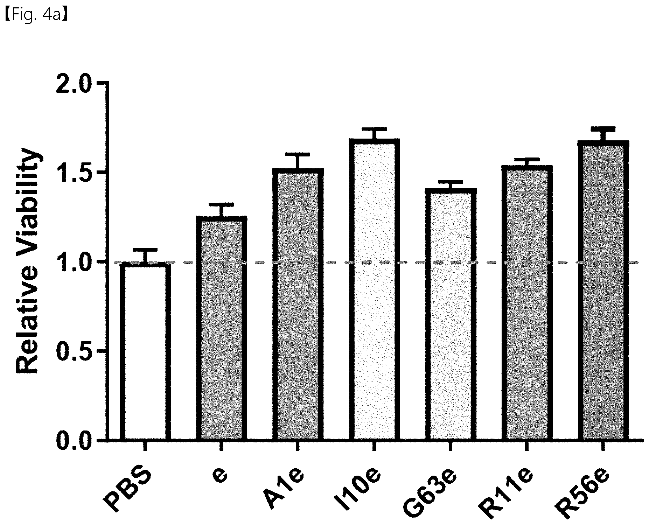

The pharmaceutical composition according to the present disclosure has excellent palliative, suppressive, prophylactic, or therapeutic effects on a vascular disease. In an exemplary embodiment, vascular endothelial fibroblasts or vascular endothelial fibroblasts damaged by hydrogen peroxide were observed to improve in proliferative capacity and viability and increase in tube length and number of node when treated with the pharmaceutical composition according to the present disclosure, compared to the control (see FIGS. 4a to 6c).

Another aspect of the present disclosure is concerned with a food composition including stem cell-derived exosomes as an active ingredient.

Since the food composition according to the present disclosure contains the same stem cell-derived exosomes as in the pharmaceutical composition stated in the foregoing, the common contents therebetween are omitted in order to avoid undue complexity of the present specification.

The food composition according to the present disclosure may contain an ingredient typically added upon the preparation of foods, for example, proteins, carbohydrates, lipids, nutrients, seasonings, and flavoring agents, but with no limitations thereto.

Examples of the carbohydrates available for the food composition according to the present disclosure include monosaccharides such as glucose, fructose, and the like, disaccharides such as maltose, sucrose, and the like, oligosaccharides, polysaccharides such as dextrin, cyclodextrin, and the like, and sugar alcohols such as xylitol, sorbitol, erythritol, and so on, but are not limited thereto.

The flavoring agent that can be used in the food composition according to the present disclosure may include a natural flavoring agent, such as thaumatin, a stevia extract, etc., and a synthetic flavoring agent, such as saccharin or aspartame, but is not limited thereto.

In the present disclosure, the food composition may be for use in palliating, preventing, or alleviating a heart disease.

In the present disclosure, the food composition may be for use in palliating, preventing, or alleviating an inflammatory disease.

In the present disclosure, the food composition may be for use in palliating, preventing, or alleviating an immune disease.

In the present disclosure, the food composition may be for use in palliating, preventing, or alleviating a fibrotic disease.

In the present disclosure, the food composition may be for use in palliating, preventing, or alleviating a vascular disease.

Another aspect of the present disclosure is drawn to a cell therapy product including induced pluripotent stem cell-derived mesenchymal stem cells.

As used herein, the term "cell therapy product" refers to a medication that is used for the purpose of therapy, diagnosis, and prophylaxis through a series of actions, including changing biological traits of cells, etc., in the method of proliferating and selecting living autologous, allogenic, or xenogeneic cells in vitro, thereby restoring the functions of cells and tissues.

In an embodiment of the present disclosure, the cell therapy product may be a stem cell therapy product.

In an embodiment of the present disclosure, the induced pluripotent stem cell-derived mesenchymal stem cells may include at least one selected from the group consisting of BxC stem cells, BxC-A1 stem cells, BxC-I10 stem cells, BxC-G63 stem cells, BxC-R11 stem cells, and BxC-R56 stem cells.

In the present disclosure, the cell therapy product may be for use in alleviating, suppressing, preventing, or treating a heart disease.

In the present disclosure, the cell therapy product may be for use in alleviating, suppressing, preventing, or treating an inflammatory disease.

In the present disclosure, the cell therapy product may be for use in alleviating, suppressing, preventing, or treating an immune disease.

In the present disclosure, the cell therapy product may be for use in alleviating, suppressing, preventing, or treating a fibrotic disease.

In the present disclosure, the cell therapy product may be for use in alleviating, suppressing, preventing, or treating a vascular disease.

Another aspect of the present disclosure is drawn to a method for preparing a composition containing exosomes isolated from stem cells or a culture thereof, the method comprising the following step:
an isolation step of isolating exosomes from stem cells or a culture thereof.

In the present disclosure, the method may be adapted to prepare a composition containing exosomes isolated from induced pluripotent stem cell-derived mesenchymal stem cells, or a culture thereof.

In an embodiment of the present disclosure, the isolation step may be carried out by isolating exosomes from induced pluripotent stem cell-derived mesenchymal stem cells or a culture thereof.

In an embodiment of the present disclosure, the stem cells may be autologous or allogenic stem cells, any type stem cells of animal origin, including humans and non-human mammals, stem cells derived from adults, or stem cells derived from embryos. For instance, the stem cells may be selected from the group consisting of embryonic stem cells, adult stem cells, induced pluripotent stem cells (iPSCs), induced pluripotent stem cell-derived mesenchymal stem cells, BxC stem cells, BxC-A1 stem cells, BxC-I10 stem cells, BxC-G63 stem cells, BxC-R11 stem cells, and BxC-R56 stem cells, but with no limitations thereto.

In the isolation step, the culture medium of the stem cells is centrifuged at 200-400xg for 5 to 20 minutes to remove remaining cells and cell debris and the supernatant is taken and subjected to centrifugation at 9,000-12,000xg for 60-80 minutes, followed by centrifuging the resulting supernatant at 90,000-120,000xg for 80-100 minutes to afford exosomes as a pellet.

In an embodiment of the present disclosure, the method may further include a pretreatment step of pretreating the induced pluripotent stem cell-derived mesenchymal stem cells with at least one selected from the group consisting of pioglitazone, phorbol 12-myristate 13-acetate, exendin-4, hyaluronic acid, and resveratrol.

In an embodiment of the present disclosure, the method may further include a selective culturing step of separating SSEA-4 (-) cells from the cultured iPSCs and culturing same to perform differentiation into BxC stem cells.

In an embodiment of the present disclosure, the method may further include an exosome production step of culturing stem cells in a cell culture medium.

The exosome production step according to the present disclosure is a process of inducing secretion or production of exosomes. In the present disclosure, the cell culture medium may be any typically used stem cell culture medium whether it is commercially available or artificially synthetic, as exemplified by , DMEM (Dulbecco's Modified Eagle's Medium), MEM (Minimal Essential Medium), BME (Basal Medium Eagle), RPMI 1640, DMEM/F-10 (Dulbecco's Modified Eagle's Medium: Nutrient Mixture F-10), DMEM/F-12 (Dulbecco's Modified Eagle's Medium: Nutrient Mixture F-12), α-MEM (a-Minimal essential Medium), G-MEM (Glasgow's Minimal Essential Medium), IMDM (Isocove's Modified Dulbecco's Medium), KnockOut DMEM, E8 (Essential 8 Medium), but with no limitations thereto.

In an embodiment of the present disclosure, the cell culture medium may further contain an ingredient such as a carbon source, a nitrogen source, a trace element, an amino acid, an antibiotic, and the like.

In an embodiment of the present disclosure, the exosome production step may further include an additional culturing step of culturing the stem cell in the presence of exosome-free fetal bovine serum (FBS).

As opposed to general FBS containing a large amount of bovine serum-derived exosomes, the exosome-free FBS in the cell culture medium can prevent the incorporation of FBS-derived exosomes into the exosomes secreted from stem cells.

Another aspect of the present disclosure is drawn to a method for preparing a composition containing exosomes, the method comprising the following steps:
a first culturing step of culturing induced pluripotent stem cells in a medium;
a selective culturing step of separating SSEA-4 (-) cells from the cultured induced pluripotent cells and culturing same to perform differentiation into BxC stem cells;
a second culturing step of culturing the BxC stem cells to perform differentiation into mesenchymal stem cells;
a pretreatment step of pretreating the mesenchymal stem cells with at least one selected from the group consisting of pioglitazone, phorbol 12-myristate 13-acetate, exendin-4, hyaluronic acid, and resveratrol;
a production step of culturing the pretreated mesenchymal stem cells to produce exosomes; and
an isolations step of isolating exosomes from the mesenchymal stem cells or a culture thereof.

In an embodiment of the present disclosure, the first culturing step may be carried out by culturing induced pluripotent stem cells for 1-10 days in a medium containing FBS and bFGF.

In an embodiment of the present disclosure, the selective culturing step may be carried out by separating SSEA-4 (-) cells from the induced pluripotent stem cells and culturing same for 1-10 days in a medium containing FBS and bFGF to perform differentiation into BxC stem cells.

In an embodiment of the present disclosure, the pretreatment step may be carried out by pretreating the mesenchymal stem cells with pioglitazone at a concentration of 0.001 to 1000 µM, 0.005 to 500 µM, 0.01 to 100 µM, 0.05 to 50 µM, 0.1 to 25 µM, 0.5 to 15 µM, 1 to 10 µM, or 2 to 6 µM, for example, at a concentration of 3 µM, but with no limitations thereto.

In an embodiment of the present disclosure, the pretreatment step may be carried out by pretreating the mesenchymal stem cells with phorbol 12-myristate 13-acetate at a concentration of 1 to 1000 nM, 5 to 500 nM, 10 to 250 nM, 10 to 100 nM, 15 to 80 nM, or 20 to 70 nM, for example, at a concentration of 50 nM, but with no limitations thereto.

In an embodiment of the present disclosure, the pretreatment step may be carried out by pretreating the mesenchymal stem cells with exendin-4 at a concentration of 0.01 to 10000 nM, 0.1 to 5000 nM, 1 to 1000 nM, 1 to 100 nM, 10 to 100 nM, 10 to 50 nM, or 10 to 30 nM, for example, at a concentration of 20 nM, but with no limitations thereto.

In an embodiment of the present disclosure, the pretreatment step may be carried out by pretreating the mesenchymal stem cells with hyaluronic acid at a concentration of 0.1 to 1000 µg/ml, 0.5 to 1000 µg/ml, 1 to 500 µg/ml, 1 to 200 µg/ml, 1 to 100 µg/ml, 1 to 80 µg/ml, 1 to 60 µg/ml, or 10 to 60 ug/ml, for example, at a concentration of 40 µg/ml, but with no limitations thereto.

In an embodiment of the present disclosure, the pretreatment step may be carried out by pretreating the mesenchymal stem cells with resveratrol at a concentration of 0.01 to 10000 nM, 0.1 to 5000 nM, 0.5 to 1000 nM, 1 to 500 nM, 1 to 100 nM, 5 to 100 nM, or 5 to 30 nM, for example, at a concentration of 10 nM, but with no limitations thereto.

In an embodiment of the present disclosure, the production step may further include an additional culturing step of culturing the mesenchymal stem cells in the presence of exosome-free fetal bovine serum (FBS). In the present disclosure, the method may be a method for preparing a pharmaceutical composition comprising exosomes isolated from induced pluripotent stem cell-derived mesenchymal stem cells or a culture thereof as an active ingredient for alleviation, suppression, prevention, or treatment of a heart disease.

In the present disclosure, the method may be a method for preparing a pharmaceutical composition comprising exosomes isolated from induced pluripotent stem cell-derived mesenchymal stem cells or a culture thereof as an active ingredient for alleviation, suppression, prevention, or treatment of an inflammatory disease.

In the present disclosure, the method may be a method for preparing a pharmaceutical composition comprising exosomes isolated from induced pluripotent stem cell-derived mesenchymal stem cells or a culture thereof as an active ingredient for alleviation, suppression, prevention, or treatment of an immune disease.

In the present disclosure, the method may be a method for preparing a pharmaceutical composition comprising exosomes isolated from induced pluripotent stem cell-derived mesenchymal stem cells or a culture thereof as an active ingredient for alleviation, suppression, prevention, or treatment of a fibrotic disease.

In the present disclosure, the method may be a method for preparing a pharmaceutical composition comprising exosomes isolated from induced pluripotent stem cell-derived mesenchymal stem cells or a culture thereof as an active ingredient for alleviation, suppression, prevention, or treatment of a vascular disease.

Another aspect of the present disclosure is drawn to a method for alleviating or treating a heart disease, the method comprising the following step:
a step of administering a pharmaceutical composition containing exosomes isolated from stem cells or a culture thereof as an active ingredient into a subject.

In an embodiment of the present disclosure, the exosomes isolated from stem cells or a culture thereof may be at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11), and BxC-R56 stem cell-derived exosomes (BxC-R56e) .

Another aspect of the present disclosure is drawn to a method for alleviating or treating an inflammatory disease, the method comprising the following step:
a step of administering a pharmaceutical composition containing exosomes isolated from stem cells or a culture thereof as an active ingredient into a subject.

In an embodiment of the present disclosure, the exosomes isolated from stem cells or a culture thereof may be at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11), and BxC-R56 stem cell-derived exosomes (BxC-R56e) .

Another aspect of the present disclosure is drawn to a method for alleviating or treating an immune disease, the method comprising the following step:
a step of administering a pharmaceutical composition containing exosomes isolated from stem cells or a culture thereof as an active ingredient into a subject.

In an embodiment of the present disclosure, the exosomes isolated from stem cells or a culture thereof may be at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11), and BxC-R56 stem cell-derived exosomes (BxC-R56e) .

Another aspect of the present disclosure is drawn to a method for alleviating or treating a fibrotic disease, the method comprising the following step:
a step of administering a pharmaceutical composition containing exosomes isolated from stem cells or a culture thereof as an active ingredient into a subject.

In an embodiment of the present disclosure, the exosomes isolated from stem cells or a culture thereof may be at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11), and BxC-R56 stem cell-derived exosomes (BxC-R56e) .

Another aspect of the present disclosure is drawn to a method for alleviating or treating a vascular disease, the method comprising the following step:
a step of administering a pharmaceutical composition containing exosomes isolated from stem cells or a culture thereof as an active ingredient into a subject.

In an embodiment of the present disclosure, the exosomes isolated from stem cells or a culture thereof may be at least one selected from the group consisting of BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11), and BxC-R56 stem cell-derived exosomes (BxC-R56e) .

Since the treatment method according to the present disclosure contemplates the same exosomes isolated from stem cells or a culture thereof as in the aforementioned composition, the common content therebetween is omitted in order to avoid undue redundancy leading to the complexity of the description.

As used herein, the term "treatment" is intended to refer to any action resulting in an alleviation in the symptoms of a disease or the beneficial alteration of the symptoms by administration of the composition according to the present disclosure.

As used herein, the term "administration" refers to provision of a substance to a patient in an appropriate manner. So long as it reaches a target tissue, any general administration route may be employed, such as oral or parenteral routes. In addition, the composition of the present disclosure can be administered by any device that is able to deliver the active ingredient to the target cells.

The term "subject", as used herein, is intended to encompass, for example, humans, monkeys, cows, horses, sheep, pigs, chickens, turkeys, quails, cats, dogs, mice, rats, rabbits, or guinea pigs, particularly humans, but with no limitations thereto.

In an embodiment of the present disclosure, the pharmaceutical composition may be administered alone or in combination of a pharmaceutically acceptable carrier that is selected in consideration of general administration routes and the standard pharmaceutical practice.

Another aspect of the present disclosure is drawn to a use of a composition comprising stem cell-derived exosomes as an active ingredient for alleviation, suppression, prevention, or treatment of a heart disease.

Another aspect of the present disclosure is drawn to a use of a composition comprising stem cell-derived exosomes as an active ingredient for alleviation, suppression, prevention, or treatment of an inflammatory disease.

Another aspect of the present disclosure is drawn to a use of a composition comprising stem cell-derived exosomes as an active ingredient for alleviation, suppression, prevention, or treatment of an immune disease.

Another aspect of the present disclosure is drawn to a use of a composition comprising stem cell-derived exosomes as an active ingredient for alleviation, suppression, prevention, or treatment of a fibrotic disease.

Another aspect of the present disclosure is drawn to a use of a composition comprising stem cell-derived exosomes as an active ingredient for alleviation, suppression, prevention, or treatment of a vascular disease.

### Advantageous Effects of Invention

The present disclosure pertains to a composition including stem cell-derived exosomes and a preparation method therefor. Having excellent effects on anti-inflammation, anti-fibrotic inhibition, vascular endothelial cell proliferation, vascularization, viability improvement, and protection and regeneration of cardiomyocytes, the composition according to the present disclosure can be used as a prophylactic or therapeutic agent for a heart disease, an inflammatory disease, an immune disease, a fibrotic disease, and a vascular disease.

### Brief Description of Drawings

FIG. 1a is a plot showing a size distribution of BxC stem cell-derived exosomes (BxC-e) according to an embodiment of the present disclosure.
FIG. 1b is an electron microscopic image showing the morphology of BxC stem cell-derived exosomes (BxC-e) according to an embodiment of the present disclosure.
FIG. 2a is a plot showing a size distribution of BxC-R11 stem cell-derived exosomes (BxC-R11e) according to an embodiment of the present disclosure.
FIG. 2b is an electron microscopic image showing the morphology of BxC-R11 stem cell-derived exosomes (BxC-R11e) according to an embodiment of the present disclosure.
FIG. 3a is a graph showing viability of induced pluripotent stem cell-derived cardiomyocytes treated with BxC stem cell-derived exosomes (BxC-e), BxC-A1 stem cell-derived exosomes (BxC-A1e), BxC-I10 stem cell-derived exosomes (BxC-I10e), BxC-G63 stem cell-derived exosomes (BxC-G63e), BxC-R11 stem cell-derived exosomes (BxC-R11e), or BxC-R56 stem cell-derived exosomes (BxC-R56e) according to an embodiment of the present disclosure.
FIG. 3b is a graph showing viability of induced pluripotent stem cell-derived cardiomyocytes damaged by hydrogen peroxide and treated with BxC-e exosomes, BxC-A1e exosomes, BxC-G63e exosomes, BxC-R11e exosomes, and BxC-R56e exosomes according to an embodiment of the present disclosure.
FIG. 3c is a view showing levels of reactive oxygen species in induced pluripotent stem cell-derived cardiomyocytes damaged by hydrogen peroxide and treated with BxC-e exosomes or BxC-R11e exosomes according to an embodiment of the present disclosure.
FIG. 3d is a graph showing levels of reactive oxygen species in induced pluripotent stem cell-derived cardiomyocytes damaged by hydrogen peroxide and treated with BxC-e exosomes or BxC-R11e exosomes according to an embodiment of the present disclosure.
FIG. 3e is a graph of LDH (lactate dehydrogenase) levels indicating degrees of damage in cardiomyocytes after iPSC-derived cardiomyocytes damaged by hydrogen peroxide are treated with BxC-e exosomes, BxC-A1e exosomes, BxC-G63e exosomes, BxC-R11e exosomes, or BxC-R56e exosomes according to an embodiment of the present disclosure.
FIG. 4a is a graph of proliferative rates of vascular endothelial cells treated with PBS and BxC-e exosomes, BxC-A1e exosomes, BxC-I10e exosomes, BxC-G63e exosomes, BxC-R11e exosomes, or BxC-R56e exosomes according to an embodiment of the present disclosure.
FIG. 4b is a graph of proliferative rates of vascular endothelial cells damaged by hydrogen peroxide (H₂O₂) and treated with PBS and BxC-e exosomes, BxC-A1e exosomes, BxC-I10e exosomes, BxC-G63e exosomes, BxC-R11e exosomes, or BxC-R56e exosomes.
FIG. 5a is a photographic image of the human umbilical cord-derived vascular endothelial cell line HUVEC treated with PBS.
FIG. 5b is a photographic image of the human umbilical cord-derived vascular endothelial cell line HUVEC treated with BxC-e exosomes.
FIG. 5c is a photographic image of the human umbilical cord-derived vascular endothelial cell line HUVEC treated with BxC-A1e exosomes.
FIG. 5d is a photographic image of the human umbilical cord-derived vascular endothelial cell line HUVEC treated with BxC-I10e exosomes.
FIG. 5e is a photographic image of the human umbilical cord-derived vascular endothelial cell line HUVEC treated with BxC-G63e exosomes.
FIG. 5f is a photographic image of the human umbilical cord-derived vascular endothelial cell line HUVEC treated with BxC-R11e exosomes.
FIG. 5g is a photographic image of the human umbilical cord-derived vascular endothelial cell line HUVEC treated with BxC-R56e exosomes.
FIG. 6a is a photographic image of the human umbilical cord-derived vascular endothelial cell line HUVEC treated with PBS, BxC-e exosomes, BxC-A1e exosomes, BxC-I10e exosomes, BxC-G63e exosomes, BxC-R11e exosomes, and BxC-R56e exosomes, showing tube lengths of vascular endothelial cells.
FIG. 6b is a photographic image of the human umbilical cord-derived vascular endothelial cell line HUVEC treated with PBS, BxC-e exosomes, BxC-A1e exosomes, BxC-I10e exosomes, BxC-G63e exosomes, BxC-R11e exosomes, and BxC-R56e exosomes, showing numbers of tube connecting nodes therein.
FIG. 6c is a photographic image of the human umbilical cord-derived vascular endothelial cell line HUVEC treated with PBS, BxC-e exosomes, BxC-A1e exosomes, BxC-I10e exosomes, BxC-G63e exosomes, BxC-R11e exosomes, and BxC-R56e exosomes, showing numbers of tubes therein.
FIG. 7a is a graph of mRNA expression levels of TNF-α in the human monocyte cell line THP-1 cultured in media containing PBS or BxC-e exosomes.
FIG. 7b is a graph of mRNA expression levels of IL-1β in the human monocyte cell line THP-1 cultured in media containing PBS or BxC-e exosomes.
FIG. 7c is a graph of mRNA expression levels of IL-6 in the human monocyte cell line THP-1 cultured in media containing PBS or BxC-e exosomes.
FIG. 8a is a graph of mRNA expression levels of TNF-α in the human monocyte cell line THP-1 cultured in media containing PBS or BxC-R11e exosomes.
FIG. 8b is a graph of mRNA expression levels of IL-1β in the human monocyte cell line THP-1 cultured in media containing PBS or BxC-R11e exosomes.
FIG. 8c is a graph of mRNA expression levels of IL-6 in the human monocyte cell line THP-1 cultured in media containing PBS or BxC-R11e exosomes.
FIG. 9a is a graph of mRNA expression levels of CD206 in the human monocyte cell line THP-1 cultured in media containing PBS or BxC-R11e exosomes.
FIG. 9b is a graph of mRNA expression levels of ARG-1 in the human monocyte cell line THP-1 cultured in media containing PBS or BxC-R11e exosomes.
FIG. 9c is a graph of mRNA expression levels of IL-10 in the human monocyte cell line THP-1 cultured in media containing PBS or BxC-R11e exosomes.
FIG. 10a is a graph of mRNA expression levels of α-SMA in the mouse embryonic fibroblast cell line CF-1 cultured in media containing PBS, BxC-e, or BxC-R11e exosomes, as measured by PCR.
FIG. 10b is a graph of mRNA expression levels of CTGF in the mouse embryonic fibroblast cell line CF-1 cultured in media containing PBS, BxC-e, or BxC-R11e exosomes, as measured by PCR.
FIG. 11 is a graph of expression levels of α-SMA, CTGF, and b-actin in the mouse embryonic fibroblast cell line CF-1 cultured in media containing PBS, BxC-e, or BxC-R11e exosomes, as measured by SDS-PAGE.
FIG. 12a is a graph of expression levels of α -SMA in the mouse embryonic fibroblast cell line CF-1 cultured in media containing PBS, BxC-e, or BxC-R11e exosomes, as measured by SDS-PAGE.
FIG. 12b is a graph of expression levels of CTGF in the mouse embryonic fibroblast cell line CF-1 cultured in media containing PBS, BxC-e, or BxC-R11e exosomes, as measured by SDS-PAGE.
FIG. 13a is a graph of expression levels of cTnT in iPSC-derived cardiomyocytes cultured in media containing PBS, BxC-e, or BxC-R11e exosomes, as measured by PCR.
FIG. 13b is a graph of expression levels of hERG in iPSC-derived cardiomyocytes cultured in media containing PBS, BxC-e, or BxC-R11e exosomes, as measured by PCR.
FIG. 13c is a graph of expression levels of Nav 1.7 in iPSC-derived cardiomyocytes cultured in media containing PBS, BxC-e, or BxC-R11e exosomes, as measured by PCR.
FIG. 13d is a graph of expression levels of MYH7 in iPSC-derived cardiomyocytes cultured in media containing PBS, BxC-e, or BxC-R11e exosomes, as measured by PCR.

### Best Mode for Carrying out the Invention

Pharmaceutical composition, for alleviation, suppression, prevention, or treatment of a heart disease, comprising at least one selected from the group consisting of BxC stem cell-derived exosomes, BxC-A1 stem cell-derived exosomes, BxC-I10 stem cell-derived exosomes, BxC-G63 stem cell-derived exosomes, BxC-R11 stem cell-derived exosomes, and BxC-R56 stem cell-derived exosomes.

### Mode for Carrying out the Invention

Below, a better understanding of the present disclosure may be obtained through the following examples which are set forth to illustrate, but are not to be construed to limit the present disclosure.

### EXAMPLE 1: Culturing of Induced Pluripotent Stem Cell-Derived Mesenchymal Stem Cells

Induced pluripotent stem cells (iPSCs) were cultured for 7 days in DMEM supplemented with 10% fetal bovine serum (FBS) and 10 ng/ml bFGF. Then, SSEA-4(-) cells that do not express stage-specific embryonic antigen 4 (SSEA-4) on the surface thereof were separated by FACS analysis from the cultured iPSCs to obtain progenitor cells of iPSC-derived mesenchymal stem cells. Subsequently, the separated SSEA-4(-) cells were passaged and cultured for an additional seven days in DMEM supplemented with 10% FBS and 10 ng/ml bFGF to afford BxC stem cells.

Thereafter, the BxC stem cells were further cultured in a culture medium containing high glucose DMEM (Gibco, USA), 10% FBS (HyClone, USA), and 1% MEM Non-Essential Amino Acids Solution (100X, Gibco, USA) to perform complete differentiation into iPSC-derived mesenchymal stem cells.

### EXAMPLE 2: Isolation and Characterization of Exosome (BxC-e) Derived from iPSC-Derived Mesenchymal Stem Cells

### 2-1. Isolation of BxC-e exosome

The culture medium of the iPSC-derived mesenchymal stem cells cultured in Example 1 was harvested and centrifuged at 300xg for 10 minutes to remove remaining cells and cell debris. The supernatant thus obtained was filtered through a 0.22-µm filter and subjected to centrifugation at 10,000xg and 4°C for 70 minutes in a high-speed centrifuge.

Subsequently, the resulting supernatant was again centrifuged at 100,000xg and 4°C for 90 minutes, using an ultracentrifuge. Afterward, the resulting supernatant was removed to obtain exosomes as a pellet. The exosomes were diluted in PBS (phosphate bufferd saline) to obtain isolated iPSC-derived mesenchymal stem cell-derived exosomes (hereinafter referred to as "BxC-e exosomes"), which were used in the subsequent experiments.

### 2-2. Characterization of BxC-e exosome

The BxC-e exosomes isolated in Example 2-1 were examined for size distribution using nanoparticle tracking assay (NanoSight NS300, Malvern Panalytical) and for morphology using an electron microscope.

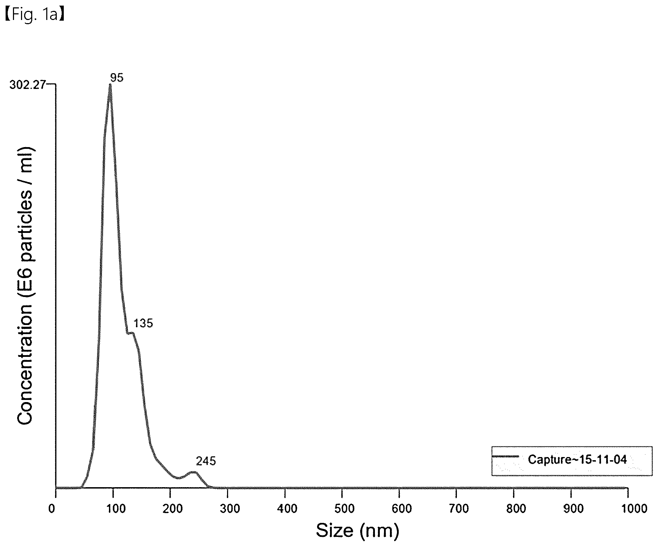

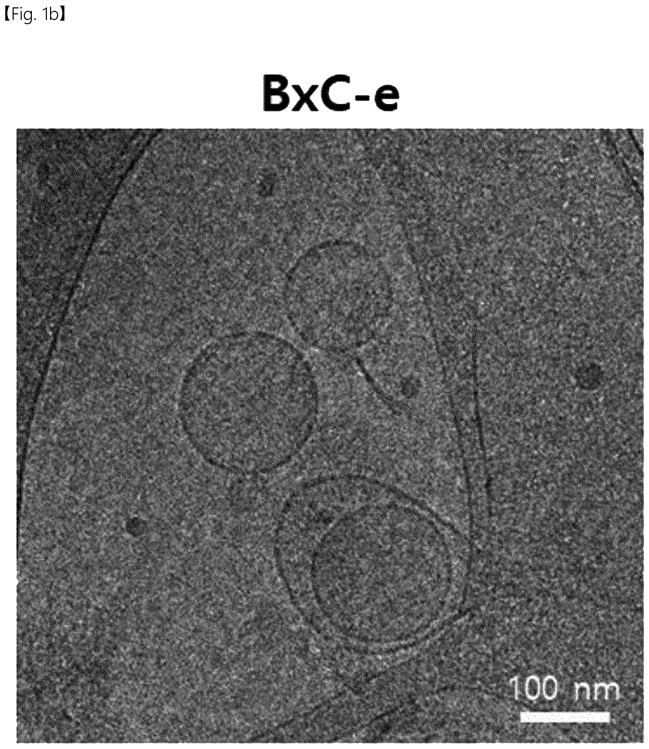

The results are depicted in FIGS. 1a and 1b. As can be seen, BxC-e exosomes were observed to have the characteristics of exosomes themselves.

### EXAMPLE 3: Isolation of Exosomes from iPSC-Derived Mesenchymal Stem Cells According to Pretreatment Substance

### 3-1. Isolation of hyaluronic acid-pretreated exosomes (BxC-R11e)

The iPSC-derived mesenchymal stem cells prepared in Example 1 were cultured for 24 hours in high-glucose DMEM medium supplemented with 10% fetal bovine serum, 1% MEM non-essential amino acids solution, and 40 ug/ml hyaluronic acid to prepare iPSC-derived mesenchymal stem cells (BxC-R11 stem cells) pretreated with hyaluronic acid.

After completion of the culturing, the BxC-R11 stem cells were washed and cultured for an additional 72 hours in a culture medium supplemented with 10% exosome-free FBS.

After 72 hours of incubation, the pretreated culture medium was harvested and centrifuged at 300xg for 10 minutes to remove remaining cells and cell debris. Subsequently, the supernatant was taken, filtered through a 0.22-pm filter, and centrifuged at 10,000xg and 4°C for 70 minutes, using a high-speed centrifuge. Then, the supernatant thus formed was taken and subjected to ultracentrifugation at 100,000xg and 4°C for 90 minutes. After removal of the resulting supernatant, the exosomes in the form of a pellet were diluted in PBS to isolate hyaluronic acid-pretreated exosomes (hereinafter referred to as " BxC-R11e exosomes"), which were used in the subsequent experiments.

### 3-2. Isolation of pioglitazone-pretreated exosomes (BxC-Ale)

The iPSC-derived mesenchymal stem cells prepared in Example 1 were cultured for 24 hours in high-glucose DMEM medium supplemented with 10% fetal bovine serum, 1% MEM non-essential amino acids solution, and 3 µM pioglitazone (Sigma, USA) to prepare iPSC-derived mesenchymal stem cells (BxC-R11 stem cells) pretreated with pioglitazone.

After completion of the culturing, the BxC-A1 stem cells were washed and cultured for an additional 72 hours in a culture medium supplemented with 10% exosome-free FBS.

After 72 hours of incubation, the pretreated culture medium was harvested and centrifuged at 300xg for 10 minutes to remove remaining cells and cell debris. Subsequently, the supernatant was taken, filtered through a 0.22-pm filter, and centrifuged at 10,000xg and 4°C for 70 minutes, using a high-speed centrifuge. Then, the supernatant thus formed was taken and subjected to ultracentrifugation at 100,000xg and 4°C for 90 minutes. After removal of the resulting supernatant, the exosomes in the form of a pellet were diluted in PBS to isolate pioglitazone-pretreated exosomes (hereinafter referred to as "BxC-A1e exosomes"), which were used in the subsequent experiments.

### 3-3. Isolation of phorbol 12-myristate 13-acetate-pretreated exosomes (BxC-I10e)

The iPSC-derived mesenchymal stem cells prepared in Example 1 were cultured for 24 hours in high-glucose DMEM medium supplemented with 10% fetal bovine serum, 1% MEM non-essential amino acids solution, and 50 nM phorbol 12-myristate 13-acetate (PMA, Sigma, USA) to prepare iPSC-derived mesenchymal stem cells (BxC-I10 stem cells) pretreated with phorbol 12-myristate 13-acetate.

After completion of the culturing, the BxC-I10 stem cells were washed and cultured for an additional 72 hours in a culture medium supplemented with 10% exosome-free FBS.

After 72 hours of incubation, the pretreated culture medium was harvested and centrifuged at 300xg for 10 minutes to remove remaining cells and cell debris. Subsequently, the supernatant was taken, filtered through a 0.22-pm filter, and centrifuged at 10,000xg and 4°C for 70 minutes, using a high-speed centrifuge. Then, the supernatant thus formed was taken and subjected to ultracentrifugation at 100,000xg and 4°C for 90 minutes. After removal of the resulting supernatant, the exosomes in the form of a pellet were diluted in PBS to isolate phorbol 12-myristate 13-acetate-pretreated exosomes (hereinafter referred to as "BxC-I10e exosomes"), which were used in the subsequent experiments.

### 3-4. Isolation of exendin-4-pretreated exosomes (BxC-G63e)

The iPSC-derived mesenchymal stem cells prepared in Example 1 were cultured for 24 hours in high-glucose DMEM medium supplemented with 10% fetal bovine serum, 1% MEM non-essential amino acids solution, and 20 nM exendin-4 (Sigma, USA) to prepare iPSC-derived mesenchymal stem cells (BxC-G63 stem cells) pretreated with exendin-4.

After completion of the culturing, the BxC-G63 stem cells were washed and cultured for an additional 72 hours in a culture medium supplemented with 10% exosome-free FBS.

After 72 hours of incubation, the pretreated culture medium was harvested and centrifuged at 300xg for 10 minutes to remove remaining cells and cell debris. Subsequently, the supernatant was taken, filtered through a 0.22-pm filter, and centrifuged at 10,000xg and 4°C for 70 minutes, using a high-speed centrifuge. Then, the supernatant thus formed was taken and subjected to ultracentrifugation at 100,000xg and 4°C for 90 minutes. After removal of the resulting supernatant, the exosomes in the form of a pellet were diluted in PBS to isolate exendin-4-pretreated exosomes (hereinafter referred to as "BxC-G63e exosomes"), which were used in the subsequent experiments.

### 3-5. Isolation of resveratrol-pretreated exosomes (BxC-R56e)

The iPSC-derived mesenchymal stem cells prepared in Example 1 were cultured for 24 hours in high-glucose DMEM medium supplemented with 10% fetal bovine serum, 1% MEM non-essential amino acids solution, and 10 nM resveratrol (Sigma, USA) to prepare iPSC-derived mesenchymal stem cells (BxC-R56 stem cells) pretreated with resveratrol.

After completion of the culturing, the BxC-R56 stem cells were washed and cultured for an additional 72 hours in a culture medium supplemented with 10% exosome-free FBS.

After 72 hours of incubation, the pretreated culture medium was harvested and centrifuged at 300xg for 10 minutes to remove remaining cells and cell debris. Subsequently, the supernatant was taken, filtered through a 0.22-pm filter, and centrifuged at 10,000xg and 4°C for 70 minutes, using a high-speed centrifuge. Then, the supernatant thus formed was taken and subjected to ultracentrifugation at 100,000xg and 4°C for 90 minutes. After removal of the resulting supernatant, the exosomes in the form of a pellet were diluted in PBS to isolate resveratrol-pretreated exosomes (hereinafter referred to as "BxC-R56e exosomes"), which were used in the subsequent experiments.

### EXAMPLE 4: Characterization of Hyaluronic Acid-Pretreated Exosomes (BxC-R11e)

The BxC-R11e exosomes isolated in Example 3-1 were examined for size distribution using nanoparticle tracking assay (NanoSight NS300, Malvern Panalytical) and for morphology using an electron microscope.

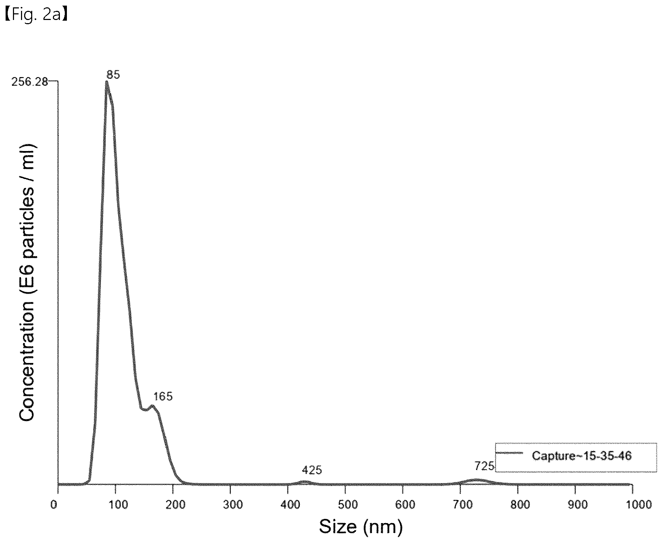

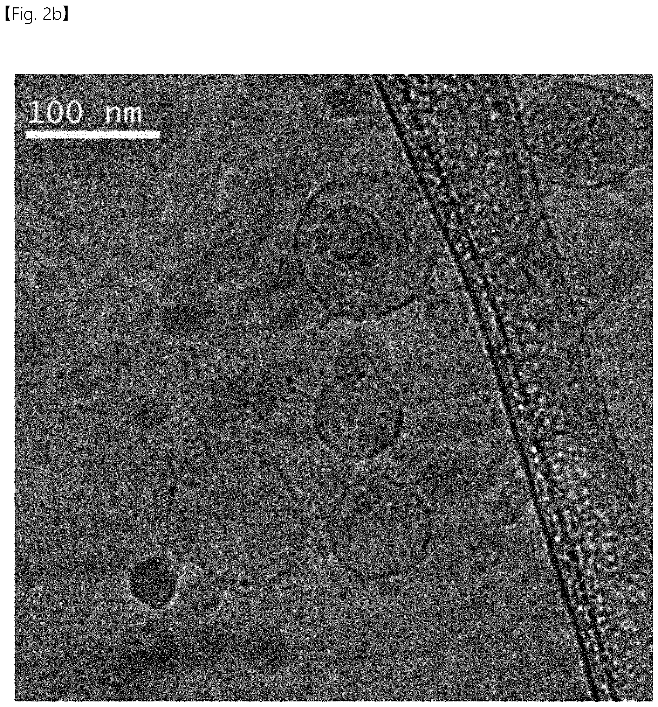

The results are depicted in FIGS. 2a and 2b. As can be seen, BxC-R11e exosomes were observed to have the characteristics of exosomes themselves.

### EXAMPLE 5: Identification of Exosome-Specific Marker on BxC-e, BxC-R11e, BxC-Ale, BxC-I10e, BxC-G63e, and BxC-R56e Exosomes

BxC-e, BxC-R11e, BxC-A1e, BxC-I10e, BxC-G63e, and BxC-R56e exosomes isolated in Examples 2 and 3 were examined for exosome-specific markers.

Twenty µL of each of BxC-e, BxC-R11e, BxC-A1e, BxC-I10e, BxC-G63e, and BxC-R56e exosomes was introduced into a tube to which 100 µL of a MACSPlex buffer (Miltenyi Biotec, Germany) was added to form a total volume of 120 µL. MACSPlex Exosome Capture Beads (Miltenyi Biotec, Germany) were resuspended by voltexing, and then added in an amount of 15 µL to each tube containing the exosomes. Each tube was incubated at room temperature for 16 hours while being shaken on an orbital shaker. The addition of 500µL of a MACSPlex buffer (Miltenyi Biotec, Germany) was followed by centrifugation at 3000xg for 5 minutes. After centrifugation, 500 µL of the supernatant in each tube was decanted and 5 µL of MACSPlex Exosome Detection Reagent (Miltenyi Biotec, Germany) was added to each tube and mixed. Then, the tubes were incubated at room temperature for 1 hour. To each tube was added 500 µL of a MACSPlex Buffer (Miltenyi Biotec, Germany), followed by centrifugation at 3000xg for 5 minutes. After removal of 500 µL of the supernatant from each tube, 500 µL of a MACSPlex Buffer was added again to each tube. Thereafter, the tubes were incubated for 15 minutes and centrifuged at 3000xg for 5 minutes. Following removal of 500 µL of the supernatant, the expression of exosome-specific markers was examined by flow cytometry. The results are summarized in Table 1.

**TABLE 1**

| Exosome-specific marker | Exosome | | | | | |
|---|---|---|---|---|---|---|
| | BxC-e | BxC-A1e | BxC-I10e | BxC-G63e | BxC-R11e | BxC-R56e |
| CD9 | 95% | 100% | 100% | 96% | 100% | 100% |
| CD63 | 100% | 100% | 100% | 100% | 100% | 100% |
| CD81 | 100% | 100% | 100% | 100% | 100% | 100% |

As understood from the data of Table 1, all of BxC-e, BxC-R11e, BxC-A1e, BxC-I10e, BxC-G63e, and BxC-R56e exosomes isolated in Examples 2 and 3 expressed exosome-specific markers at a level of 95% or higher. Accordingly, all the exosomes isolated in Examples 2 and 3 were observed to have characteristics of exosomes.

### EXAMPLE 6: Regenerative Effect on Cardiomyocyte

### 6-1. Culturing of cardiomyocytes differentiated from iPSC

Matrigel hESC-qualified Matrix (BD Bioscicence, 354277) was diluted at a ratio of 1:100 in DMEM/F12+GlutaMAX^{™} (GIBCO, 10565-018) and the dilution was coated at a density of 0.5 ml/well onto 12-well cell culture plates (SPL, 30012) by incubation at 37°C for one hour. Induced pluripotent stem cells were cultured to a confluence of 80-90 % in 75T flasks. The cells were washed twice with 5 ml of DPBS free of calcium and magnesium (HYCLONE, SH30028.02), followed by incubation with 4 ml of Tryple^{™} Express (1X), no phenol red (GIBCO, 12604-021) at 37°C for 4 minutes. The cells were suspended in a DMEM/F12 + GlutaMAX^{™} supplement containing 10% FBS (GIBCO, 16000-044) and collected by centrifugation at 1000 rpm for 3 min. The supernatant was decanted and the cells were suspended 10 times in 1 ml of mTeSRTM1 (STEMCELL Technologies, 85850) and counted. The cells were seeded at a density of 0.5×10⁶/well in 1 ml of mTeSRTM1 and incubated to reach a confluence of 90-95% while the mTeSRTM1 medium was replaced every day for 2-4 days with a fresh one. The induced pluripotent stem cells with a confluence of 90-95% were cultured in 2 ml of RPMI1640 Medium (GIBCO, 11875-093) + B-27TM Supplement, minus insulin (GIBCO, A1895601) supplemented with CHIR99021 (Tocris, 4423) 6 µM/ml + BMP4 (ProSpec, CYT-361) 10ng/ml + StemBead Activin-A (Stem Culture, SBAC5) 10 ng/ml. After 48 hours of incubation, the cells were washed once with dPBS 1 ml/well and the medium was replaced with 2 ml of RPMI1640 Medium + minus insulin supplement containing XAV939 (Sigma, X3004) 10 µM/ml + L-Ascorbic acid (Sigma, A5960) 50 ug/ml. After 48 hours of incubation, the cells were washed once with DPBS 1 ml/well, and the medium was changed again with 2 ml RPMI1640 Medium + minus insulin supplement containing 50 µg/ml ascorbic acid. After additional 48 hours of incubation, the cells were washed with dPBS 1 ml/well and then incubated in 2 ml of RPMI1640 Medium + B-27TM Supplement, minus vitamin A (GIBCO, 12587010). After 24 hours, the cultured cells were observed to beat to demonstrate complete differentiation into cardiomyocytes.

The differentiated cardiomyocytes observed to beat were seeded on 12-well plates coated at 5 ml/well per 10 cm with Matrigel hESC-qualified Matrix diluted at a ratio of 1:100 in DMEM/F12+GlutaMAX^{™} and incubated at 37°C for one hour. The cells were washed twice with 5 ml of dPBS and incubated with 4 ml of Tryple at 37°C for 4 min. The cells were harvested in DMEM/F12 supplemented with 10% FBS and centrifuged at 1000 rpm for 3 min. After removal of the supernatant, the cell pellet was suspended 10 times in 1 ml of RPMI1640 without vitamin A supplement and then counted. The cells were seeded at a population of 1×10⁷ cells in a 10-cm dish containing 10 ml of RPMI1640 without vitamin A supplement plus 5 µM/ml Y27632. After 24 hours, the medium was changed with 10 ml of RPMI 1640 Medium, no glucose (GIBCO, 11879-020) and the cardiomyocytes were maintained for 2-4 days while checking the purity of cardiomyocytes. When the cardiomyocytes were observed to be high in yield after 2-4 days, the medium was changed with 10 ml of RPMI1640 without vitamin A supplement. The cells were used after a restoration period of 48 hours or subjected to cell cryopreservation. When the cells were used immediately, the medium was changed every 48 hours with RPMI1640 without vitamin A supplement in the subsequent viability assay.

### 6-2. Proliferative effect on cardiomyocytes

To examine the proliferative effect of exosomes on cardiomyocytes, cardiomyocytes differentiated from induced pluripotent stem cells were seeded into 96-well plates containing RPMI1640 without vitamin A supplement and incubated at 37°C for 24 hours in a 5% CO₂ incubator. After aspiration of the culture medium, the cells were washed with DPBS, added with 100 ul of RPMI1640 containing BxC-e, BxC-R11e, BxC-A1e, BxC-I10e, BxC-G63e, or BxC-R56e at a concentration of 100 ug/ml, and then incubated at 37°C for 2 days in a 5% CO₂ incubator.

Thereafter, 10 µl of Cell Counting Kit-8 (CCK-8, Enzo) solution was added to the cell culture which was then incubated at 37°C for 2 hours in a 5% CO₂ incubator. A color change was observed and absorbance was read at 450 nm to measure proliferation rates of vascular endothelial cells. The results are depicted in FIG. 3a and summarized in Table 2.

**TABLE 2**

| | CTRL | e | A1e | G63e | R11e | R56e |
|---|---|---|---|---|---|---|
| Viability (%) of cardiomyocyte | 102.6 | 126 | 139 | 143.3 | 158 | 168.6 |

As can be seen in FIG. 3a and Table 2, BxC-e, BxC-R11e, BxC-A1e, BxC-I10e, BxC-G63e, and BxC-R56e exosomes were all observed to significantly proliferate cardiomyocytes.

### 6-3. Measurement of viability in cardiomyocytes damaged by hydrogen peroxide

To examine the proliferative effect of exosomes on cardiomyocytes, cardiomyocytes differentiated from induced pluripotent stem cells were seeded into 96-well plates containing RPMI1640 without vitamin A supplement and incubated at 37°C for 24 hours in a 5% CO₂ incubator. The medium was changed with a medium containing 500 µM hydrogen peroxide (H₂O₂, Sigma, USA), followed by incubation at 37°C for 2 hours in a 5% CO₂ incubator to impart oxidative stress to the cells.

After oxidative damage to the cells, the plates were washed with DPBS, added with 100 ul of RPMI1640 without vitamin A supplement containing BxC-e, BxC-R11e, BxC-A1e, BxC-I10e, BxC-G63e, or BxC-R56e exosomes at a concentration of 100 ug/ml, and then incubated at 37°C for 2 days in a 5% CO₂ incubator so as to examine the proliferative effects of the exosomes on cardiomyocytes.

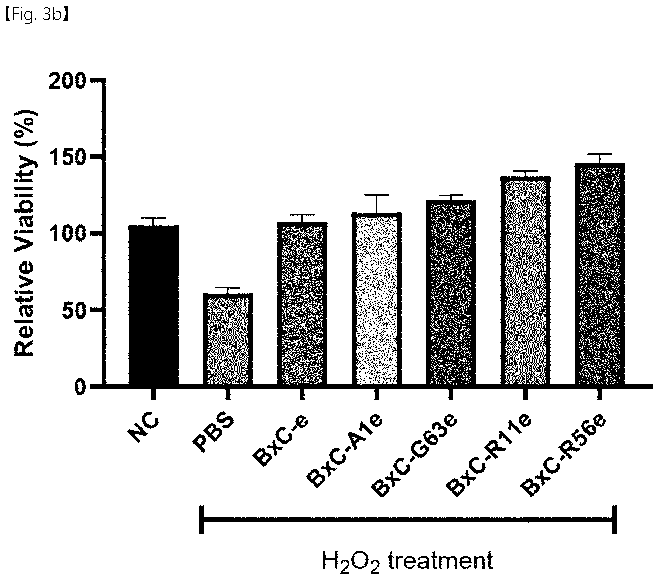

Thereafter, 10 µl of Cell Counting Kit-8 (CCK-8, Enzo) solution was added to the cell culture which was then incubated at 37°C for 2 hours in a 5% CO₂ incubator. A color change was observed and absorbance was read at 450 nm to measure viability of vascular endothelial cells. The results are depicted in FIG. 3b and summarized in Table 3.

**TABLE 3**

| | CTRL | H₂O₂ | e | A1e | G63e | R11e | R56e |
|---|---|---|---|---|---|---|---|
| Viability (%) of cardiomyocyte | 105 | 60.6 | 107.3 | 113.3 | 121.6 | 137 | 145.7 |

As can be seen in FIG. 3b and Table 3, when treated with BxC-e, BxC-R11e, BxC-A1e, BxC-I10e, BxC-G63e, or BxC-R56e exosomes, the damaged cardiomyocytes significantly increased in viability.

### 6-4. Measurement of reactive oxygen species in cardiomyocyte damaged by hydrogen peroxide

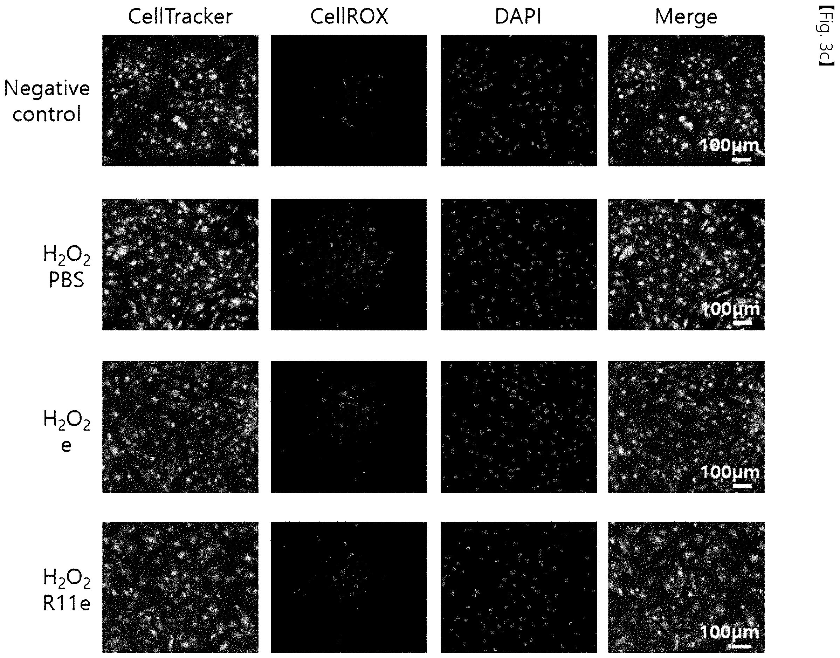

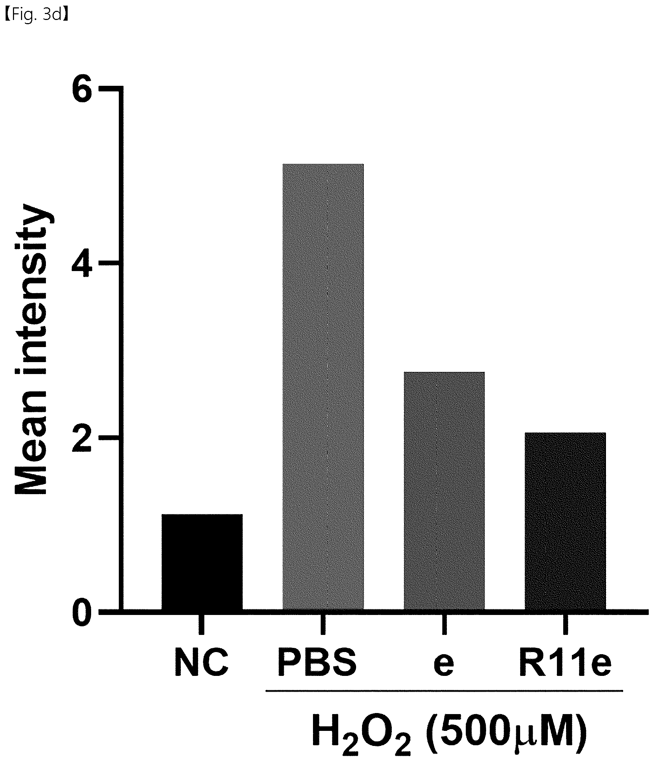

The cardiomyocytes differentiated from induced pluripotent stem cells were seeded into 24-well plates containing RPMI1640 without vitamin A supplement and then incubated at 37°C for 24 hours in a 5% CO₂ incubator. The medium was changed with a medium containing 500 µM hydrogen peroxide (H₂O₂), followed by incubation at 37°C for 24 hours in a 5% CO₂ incubator to impart oxidative stress to the cells. After oxidative damage to the cells, the medium was replaced with 300 ul of a medium containing 500 µM hydrogen peroxide (H₂O₂) and DPBS, BxC-e, BxC-R11e, BxC-A1e, BxC-I10e, BxC-G63e, or BxC-R56e at a concentration of 100 ug/ml and the cells were incubated at 37°C for 1 day in a 5% CO₂ incubator to examine the effects of the exosomes on the reactive oxygen species in the cardiomyocytes. Then, the cells were incubated in the presence of 5 µM of the reactive oxygen species measuring reagent CellROX (Invitrogen, USA) and 5 µM of CellTracker (Invitrogen, USA) at 37°C for 30 min in a 5% CO₂ incubator. Subsequently, the cells were washed with DPBS, fixed with 4% paraformaldehyde, added into NucBlue-containing DPBS, and then subjected to fluorescent microphotography. The fluorescence images are depicted in FIG. 3c. Mean intensities of fluorescence were measured and are given in FIGS. 3d and Table 4.

**TABLE 4**

| | Negative control | H₂O₂ PBS | H₂O₂ e | H₂O₂ R11e |
|---|---|---|---|---|
| Mean intensity | 1.13 | 5.14 | 2.76 | 2.06 |

As is understood from the data of FIGS. 3c to 3d and Table 4, treatment with BxC-e or BxC-R11e exosomes decreased the level of reactive oxygen species generated by H₂O₂ in the cardiomyocytes differentiated from induced pluripotent stem cells.

### 6-5. Measurement of cytotoxicity in cardiomyocytes damaged by hydrogen peroxide

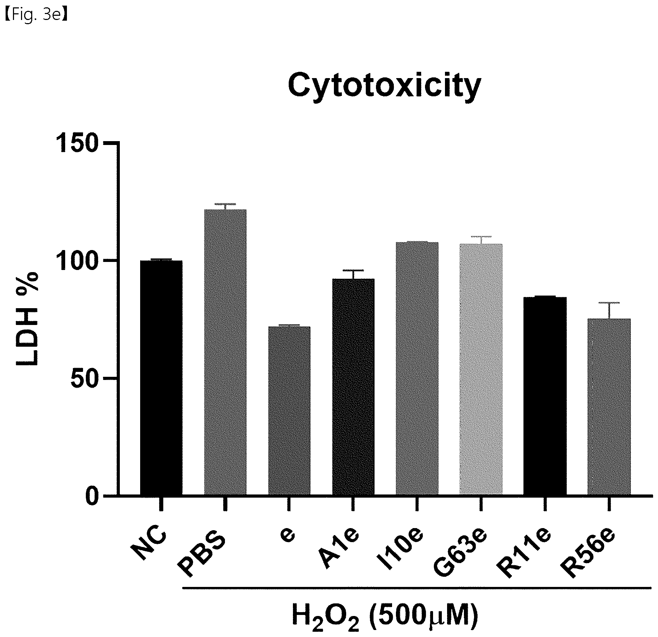

To examine the protective effect of exosomes against cytotoxicity, cardiomyocytes differentiated from induced pluripotent stem cells were seeded into 24-well plates containing RPMI1640 without vitamin A supplement and incubated at 37°C for 24 hours in a 5% CO₂ incubator. Subsequently, the medium was replaced with 300 µl of a medium containing 500 µM hydrogen peroxide (H₂O₂) and DPBS, BxC-e, BxC-R11e, BxC-A1e, BxC-I10e, BxC-G63e, or BxC-R56e at a concentration of 100 ug/ml and the cells were incubated at 37°C for 6 hours in a 5% CO₂ incubator. Then, the culture medium where the cells had been cultured was transferred into 1.5-mL tube, followed by spinning the suspended cells down. The medium was transferred in an amount of 10 µl to each well in 96-well plates. After an LDH solution (Cell cytotoxicity assay kit, BIOMAX, Korea) was added in an amount of 100 µl to each well, incubation was carried out at room temperature for 30 min. Absorbance was read at 450 nm, and the measurements are depicted in FIG. 3e and summarized in Table 5.

**TABLE 5**

| | Negative control | H₂O₂ | H₂O₂ | H₂O₂ | H₂O₂ | H₂O₂ | H₂O₂ | H₂O₂ |
|---|---|---|---|---|---|---|---|---|
| | | PBS | e | A1e | I10e | G63e | R11e | R56e |
| LDH % | 100.0 | 121.7 | 72.1 | 92.5 | 107.8 | 107.2 | 84.4 | 75.5 |

As can be seen in FIG. 3e and Table 5, the H₂O₂-induced cytotoxicity was lowered in the cardiomyocytes treated with BxC-e, BxC-R11e, BxC-A1e, BxC-I10e, BxC-G63e, or BxC-R56e exosomes.

### EXAMPLE 7: Effect on Proliferation, Survival, and Formation of Vascular Endothelial Cells

### 7-1. Measurement of proliferative effect on vascular endothelial cells

To examine the proliferative effect of exosomes on vascular endothelial cells, HUVEC (human umbilical vein endothelial cells, LONZA, Switzerland), which is human umbilical cord-derived vascular endothelial cell line, was seeded into 96-well plates containing a 2% FBS-supplemented EGM-2 medium (Lonza, Switzerland) and incubated at 37°C for 24 hours in a 5% CO₂ incubator. After removal of the culture medium, the cells were washed with DPBS, added with 100 µl of an FBS-free EGM-2 medium containing BxC-e, BxC-R11e, BxC-A1e, BxC-I10e, BxC-G63e, or BxC-R56e exosomes at a concentration of 100 ug/ml, and then incubated at 37°C for 2 days in a 5% CO₂ incubator.

Thereafter, 10 µl of Cell Counting Kit-8 (CCK-8, Enzo) solution was added to the culture medium which was then incubated at 37°C for 2 hours in a 5% CO₂ incubator. A color change was observed and absorbance was read at 450 nm to measure proliferative rates of the vascular endothelial cells. The results are depicted in FIG. 4a and summarized in Table 6.

**TABLE 6**

| | PBS | e | A1e | I10e | G63e | R11e | R56e |
|---|---|---|---|---|---|---|---|
| Proliferati on rate (%) of vascular endothelial cell | 100 | 130 | 150 | 170 | 140 | 150 | 170 |

As can be seen in FIG. 4a and Table 6, BxC-e, BxC-R11e, BxC-A1e, BxC-I10e, BxC-G63e, and BxC-R56e exosomes were all observed to significantly proliferate vascular endothelial cells.

### 7-2. Measurement of viability of vascular endothelial cells damaged by hydrogen peroxide

HUVEC, which is human umbilical cord-derived vascular endothelial cell line, was seeded into 96-well plates containing a 2% FBS-supplemented EGM-2 medium and incubated at 37°C for 24 hours in a 5% CO₂ incubator. Subsequently, the medium was changed with a medium containing 500µM hydrogen peroxide (H₂O₂, Sigma, USA), followed by incubation at 37°C for 2 hours in a 5% CO₂ incubator to damage the cultured cells.

After imparting damage to the cells, the proliferative effect of the exosomes on vascular endothelial cells was examined. In this regard, the plates were washed with DPBS and added with 100 µl of an FBS-free EGM-2 medium containing BxC-e, BxC-R11e, BxC-A1e, BxC-I10e, BxC-G63e, or BxC-R56e exosomes at a concentration of 100 µg/ml before incubation at 37°C for 2 days in a 5% CO₂ incubator.

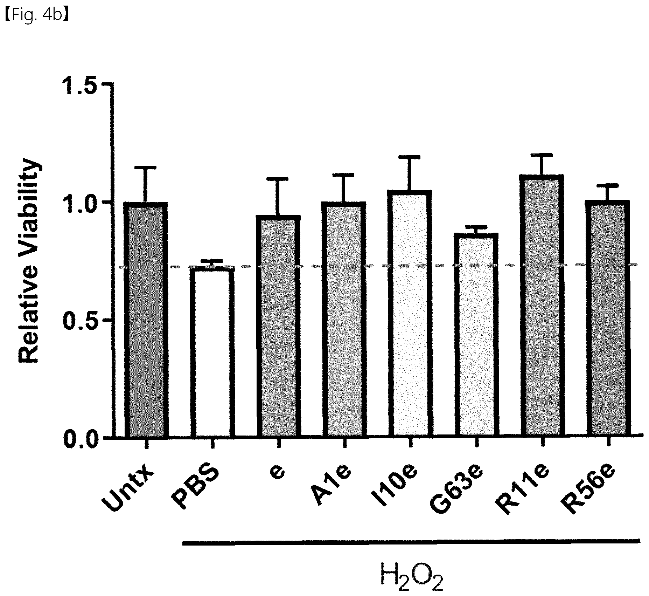

Thereafter, 10 µl of Cell Counting Kit-8 (CCK-8, Enzo) solution was added to the culture medium which was then incubated at 37°C for 2 hours in a 5% CO₂ incubator. A color change was observed and absorbance was read at 450 nm to measure viability of the vascular endothelial cells. The results are depicted in FIG. 4b and summarized in Table 7.

**TABLE 7**

| | untx | H₂O₂ | | | | | | |
|---|---|---|---|---|---|---|---|---|
| | | PBS | e | A1e | I10e | G63e | R11e | R56e |
| Viability (%) of vascular endothelial cells | 100 | 70 | 90 | 100 | 100 | 90 | 110 | 100 |

As can be seen in FIG. 4b and Table 7, when treated with BxC-e, BxC-R11e, BxC-A1e, BxC-I10e, BxC-G63e, or BxC-R56e exosomes, the damaged vascular endothelial cells significantly increased in viability.

### 7-3. Measurement of effect on vascularization

Using a cooled tip, matrigel (BD bioscience) thawed in ice was plated into 96-well plated cooled at - 20°C for one hour with care to avoid bubble formation, followed by incubation at 37°C for one hour in a 5% CO₂ incubator.

The human umbilical cord-derived vascular endothelial cell line HUVEC was seeded into the Matrigel-coated, 96-well plates containing a FBS-free EGM-2 medium. Subsequently, the medium was added with 100 ug/ml BxC-e, BxC-R11e, BxC-A1e, BxC-I10e, BxC-G63e, or BxC-R56e exosomes while PBS was added, instead of exosomes, for a control.

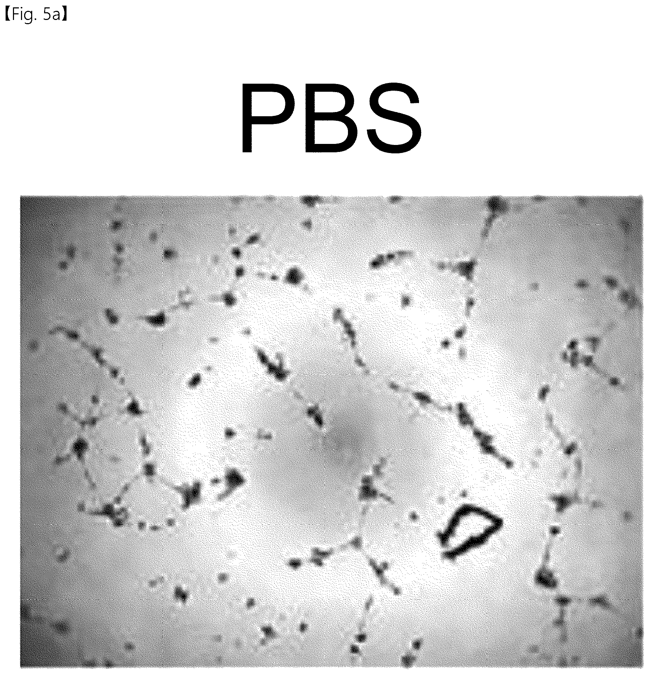

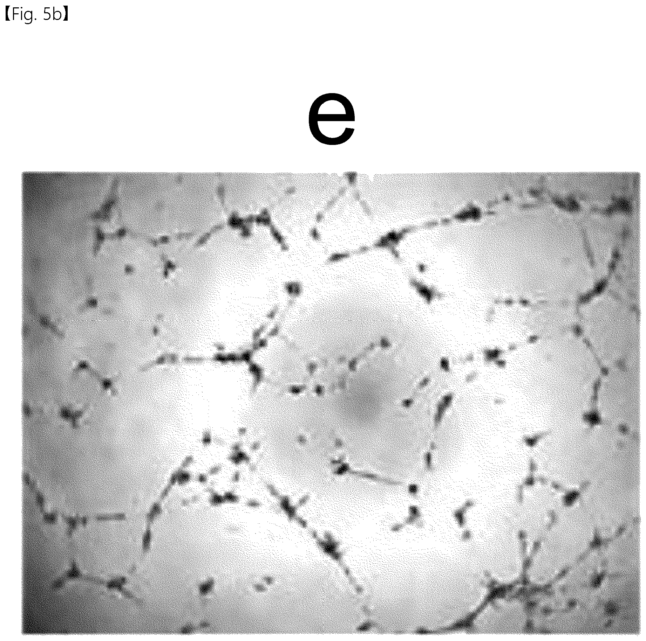

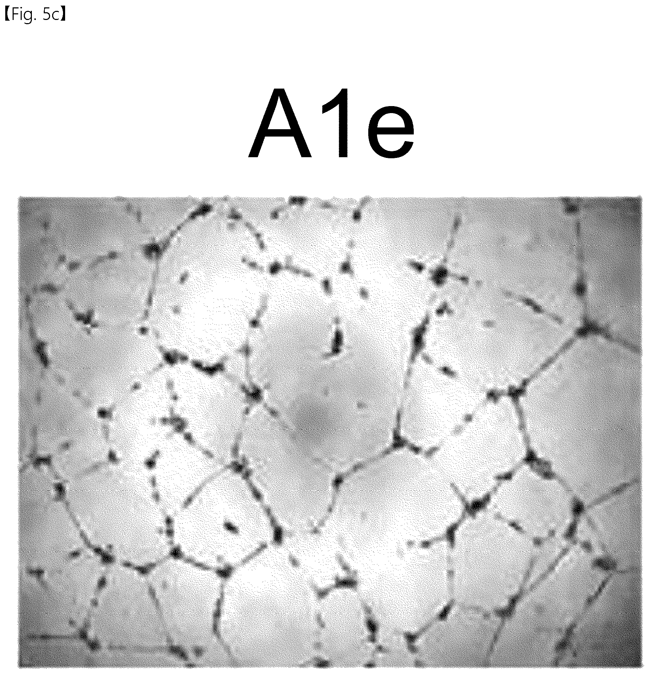

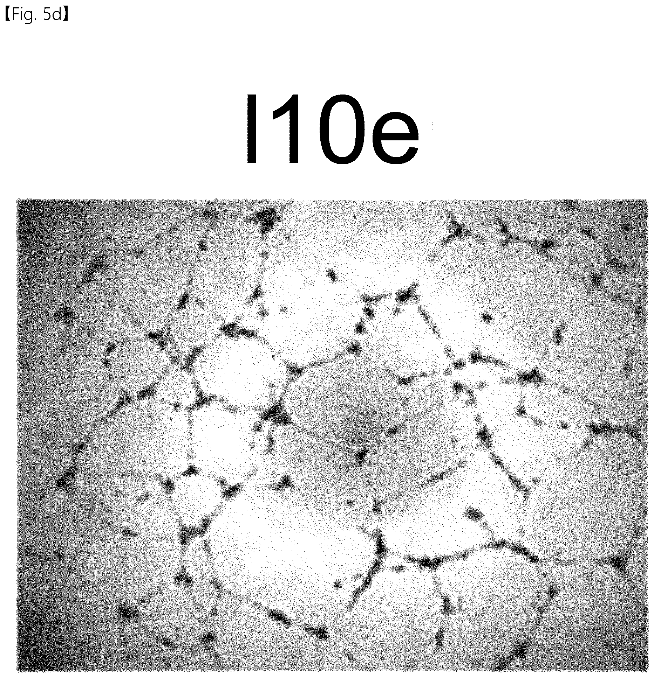

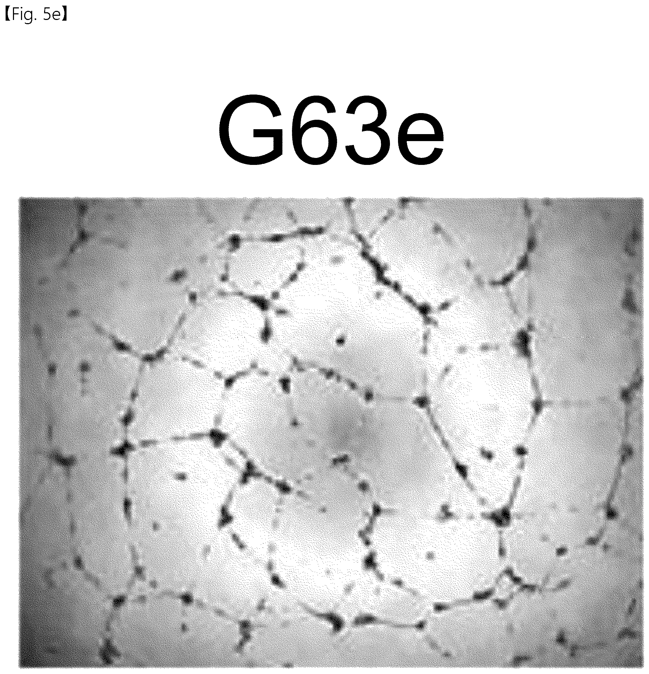

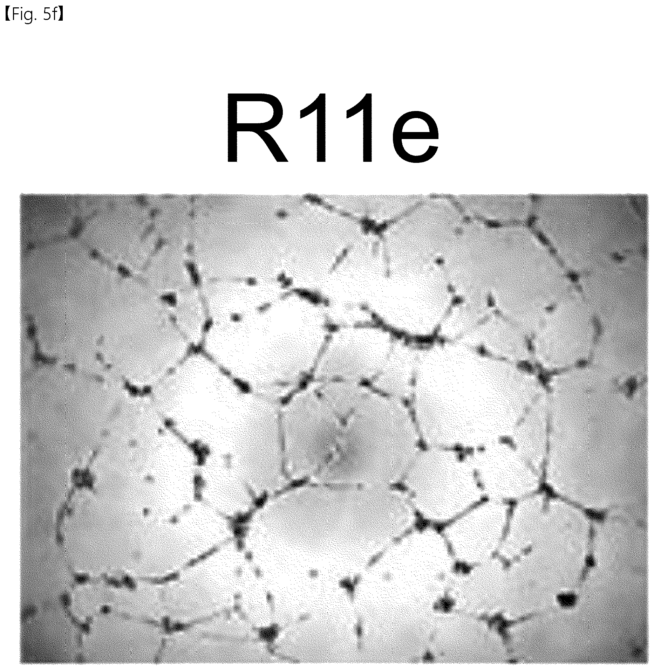

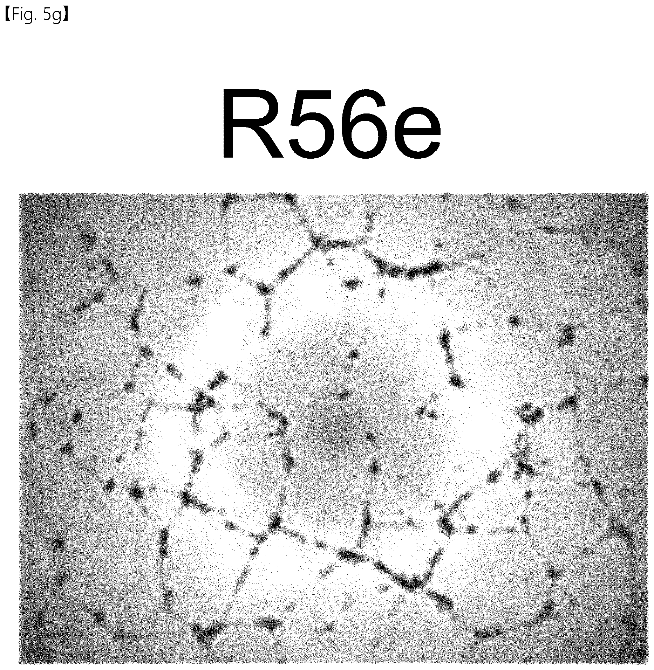

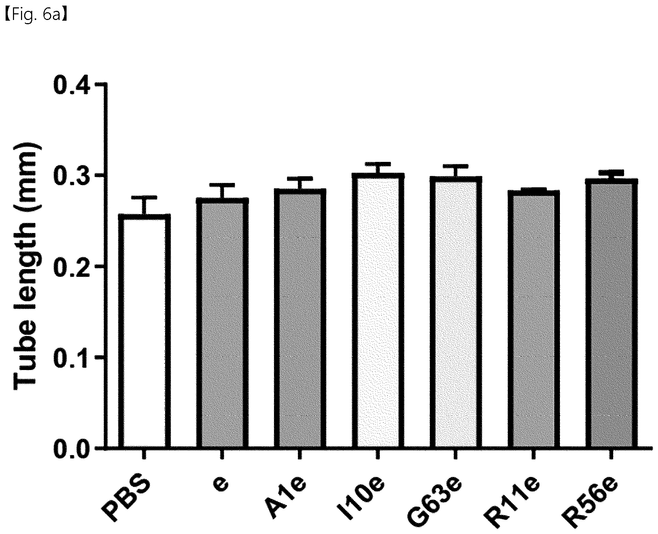

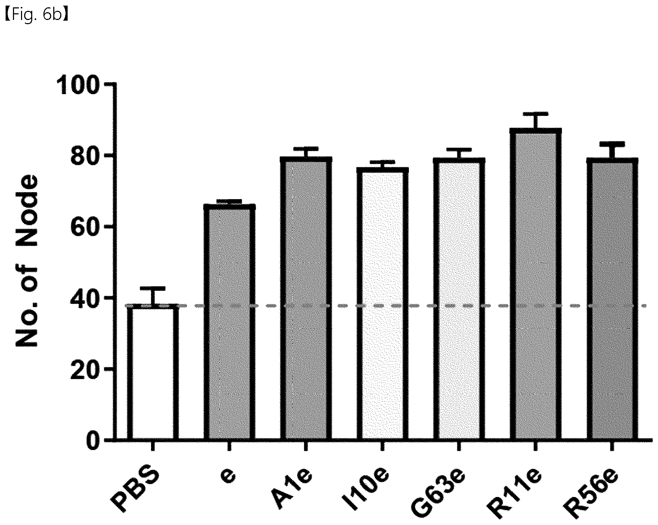

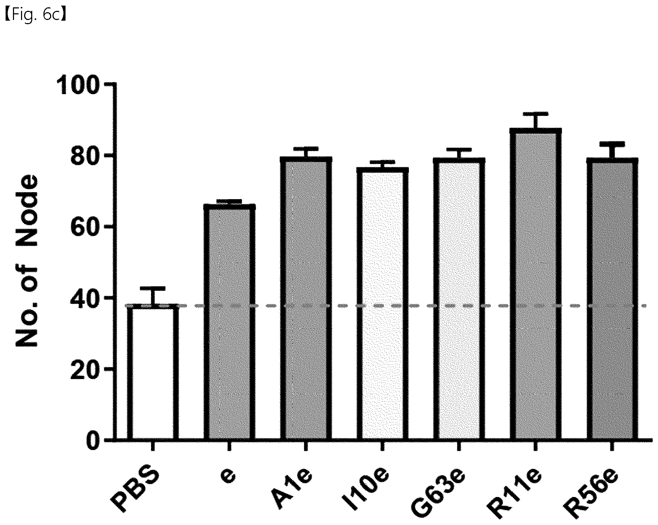

Then, the cells were incubated at 37°C within 16 hours in a 5% CO₂ incubator and photographed. The images are given in FIGS. 5a to 5g. The images were quantitatively analyzed for tube length, tube node, number of tubes, using Image J program. The data are depicted in FIGS. 6a and 6c and summarized in Tables 8 to 10.

**TABLE 8**

| | PBS | e | A1e | I10e | G63e | R11e | R56e |
|---|---|---|---|---|---|---|---|
| Tube length of vascular endothelial cell | 0.26 | 0.28 | 0.29 | 0.3 | 0.3 | 0.28 | 0.3 |

**TABLE 9**

| | PBS | e | A1e | I10e | G63e | R11e | R56e |
|---|---|---|---|---|---|---|---|
| No. of tube node of vascular endothelial cell | 38 | 66 | 80 | 77 | 79 | 88 | 80 |

**TABLE 10**

| | PBS | e | A1e | I10e | G63e | R11e | R56e |
|---|---|---|---|---|---|---|---|
| No. of vascular endothelial cell | 56 | 80 | 113 | 104 | 111 | 120 | 115 |

As can be seen in FIGS. 6a to 6c and Tables 8 to 10, BxC-e, BxC-R11e, BxC-A1e, BxC-I10e, BxC-G63e, and BxC-R56e exosomes improved tube length, number of tube node, and number of tubes in the vascular endothelial cells, demonstrating that the exosomes according to the present disclosure have an excellent effect on vascularization of vascular endothelial cells.

### EXAMPLE 8: Suppressive Effect of BxC-e Exosome on Inflammatory Macrophage

The human monocyte cell line THP-1 (ATCC, USA) was seeded into a 35-mm dish containing an RPMI1640 medium (Gibco, USA) supplemented with 1% antibiotics-antimycotics (Gibco, USA), 10% FBS (HyClone), and phorbol 12-myristate 13-acetate (PMA, 200 ng/ml, Sigma, USA) and incubated at 37°C for 2 days in a 5% CO₂ incubator.

After 48 hours of incubation, the cells were observed to adhere to the dish. Subsequently, the cells were induced to differentiate into inflammatory macrophages. In this regard, the medium was aspirated and the cells were washed with DPBS (HyClone, USA) and then incubated at 37°C for an additional 3 days in an RPMI1640 medium supplemented with lipopolysaccharides (LPS, 100ng/ml, Sigma), recombinant human interferon-gamma (INF-γ, 20ng/ml, R&D System, USA), 1% antibiotics-antimycotics, and 10% FBS in a 5% CO₂ incubator to prepare inflammatory macrophages (M1).

After 72 hours, the medium was removed and the cells were washed DPBS and incubated at 37°C for 24 hours in an RPMI1640 medium supplemented with 1% antibiotics-antimycotics and 50 ug/ml BxC-e exosomes in a 5% CO₂ incubator so as to examine an anti-inflammatory effect of the exosomes. Then, 1 ml of TRIZol reagent (Invitrogen, USA) was added to the 35-mm dish to lyse the cells. The cell lysate was added with 200 µl of chloroform (Sigma, USA), briefly vortexed, and centrifuged at 4°C and 12,000 rpm for 15 min. The supernatant thus formed was transferred into a new tube and mixed with 500 µl of isopropanol (Merck Millipore, USA). The tube was turned upside down 50 times, left on ice for 5 min, and centrifuged at 12,000 rpm and 4°C for 10 min. The supernatant was removed, and 1 ml of 70% ethanol was added to the tube to resuspend the pellet, followed by centrifugation at 12,000 rpm and 4°C for 5 min. The ethanol was aspirated, and the RNA pellet in the tube was dried before dissolution in nuclease-free water. The concentration of the RNA sample thus obtained was measured by Nanodrop absorbances at 260 nm and 280 nm and cDNA was synthesized therefrom using AccuPower CycleScript RT PreMix(dT₂₀) (K-2044, Bioneer, Korea).

Thereafter, TNF-α, IL-1β, and IL-6 were monitored for mRNA expression levels by real-time PCR using the synthesized cDNA and the primers of Table 11, and the results are given in FIGS. 7a to 7c and Tables 12 to 14.

**TABLE 11**

| SEQ ID NO: | Name | Sequencing List (5'-> 3') | Note |
|---|---|---|---|
| 1 | TNF-α_primer 1 | GAGCTGAACAATAGGCTGTTCCCA | TNF-α forward primer |
| 2 | TNF-α_primer 2 | AGAGGCTCAGCAATGAGTGACAGT | TNF-α reverse primer |
| 3 | IL-1β_primer 1 | ACAGCTGGAGAGTGTAGATCC | IL-1β forward primer |
| 4 | IL-1β_primer 2 | CTTGAGAGGTGCTGATGTACC | IL-1β reverse primer |
| 5 | IL-6_primer 1 | ACAGCCACTCACCTCTTCAG | IL-6 forward primer |
| 6 | IL-6_primer 2 | CCATCTTTTTCAGCCATCTTT | IL-6 reverse primer |

As can be seen in FIGS. 7a to 7c and Tables 12 to 14, relative expression levels of TNF-α, IL-1β, and IL-6 were measured to decrease by about 38%, about 26%, and about 35% in BxC-e exosome-treated inflammatory macrophages, respectively, compared to PBS-treated control.

**TABLE 12**

| | M0 | M1 | |
|---|---|---|---|
| | | PBS | BxC-e |
| Expression level of TNF-α | 1.0 | 7.2 | 4.5 |

**TABLE 13**

| | M0 | M1 | |
|---|---|---|---|
| | | PBS | BxC-e |
| Expression level of IL-1β | 1.0 | 5.4 | 4.0 |

**TABLE 14**

| | M0 | M1 | |
|---|---|---|---|
| | | PBS | BxC-e |
| Expression level of IL-6 | 1.0 | 4.3 | 2.8 |

The data obtained from the above experiments indicate that the BxC-e exosomes according to the present disclosure decreases levels of inflammatory cytokines expressed from inflammatory macrophages.

### EXPERIMENTAL EXAMPLE 9: Regulatory Effect of BxC-R11e Exosome on Macrophage

### 9-1. Inhibitory effect on inflammatory macrophage

The human monocyte cell line THP-1 was seeded into a 35-mm dish containing an RPMI1640 medium supplemented with 1% antibiotics-antimycotics, 10% FBS, and PMA (200 ng/ml) and incubated at 37°C for 2 days in a 5% CO₂ incubator. After 48 hours of incubation, when the cells were observed to adhere to the dish, the cells were induced to differentiate into inflammatory macrophages. In this regard, the medium was aspirated and the cells were washed with DPBS and then incubated at 37°C for an additional 3 days in an RPMI1640 medium supplemented with LPS (100ng/ml), INF-γ (20 ng/ml), 1% antibiotics-antimycotics, and 10% FBS in a 5% CO₂ incubator. After 72 hours, the medium was removed and the cells were washed DPBS and incubated at 37°C for 24 hours in an RPMI1640 medium supplemented with 1% antibiotics-antimycotics and 50 ug/ml BxC-R11e exosomes in a 5% CO₂ incubator so as to examine an anti-inflammatory effect of the exosomes. Then, 1 ml of TRIZol reagent (Invitrogen, USA) was added to the 35-mm dish to lyse the cells. The cell lysate was added with 200 µl of chloroform (Sigma, USA), briefly vortexed, and centrifuged at 4°C and 12,000 rpm for 15 min. The supernatant thus formed was transferred into a new tube and mixed with 500 µl of isopropanol (Merck Millipore, USA). The tube was turned upside down 50 times, left on ice for 5 min, and centrifuged at 12,000 rpm and 4°C for 10 min. The supernatant was removed, and 1 ml of 70% ethanol was added to the pellet in the tube, followed by centrifugation at 12,000 rpm and 4°C for 5 min. The ethanol was aspirated, and the RNA pellet in the tube was dried before dissolution in nuclease-free water. The concentration of the RNA sample thus obtained was measured by Nanodrop absorbances at 260 nm and 280 nm and cDNA was synthesized therefrom using AccuPower CycleScript RT PreMix(dT₂₀) (K-2044, Bioneer, Korea).

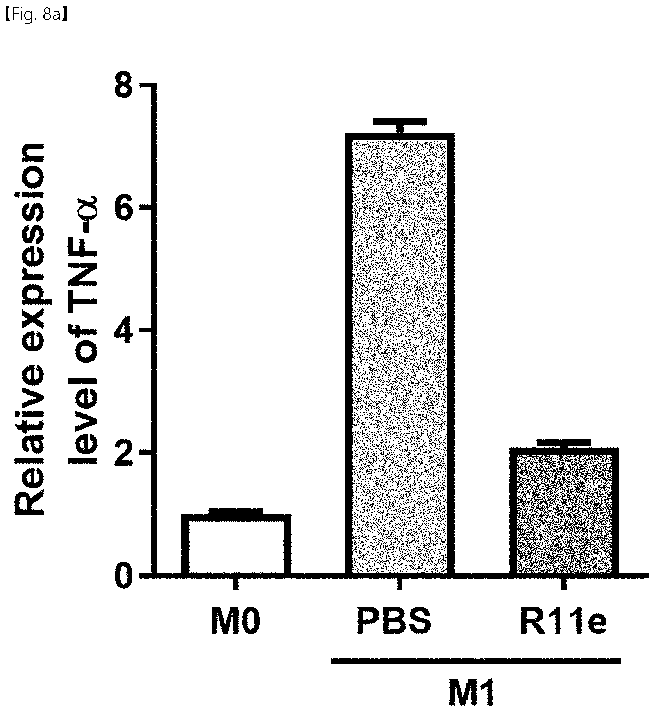

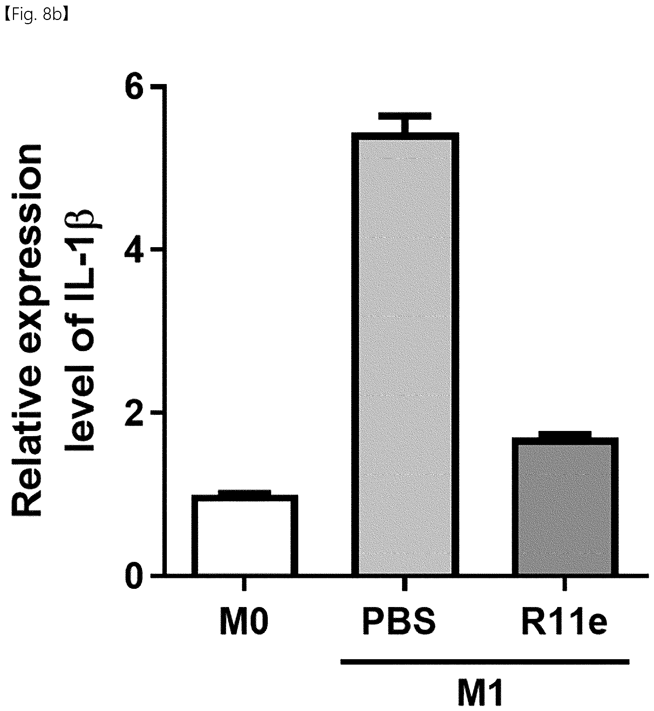

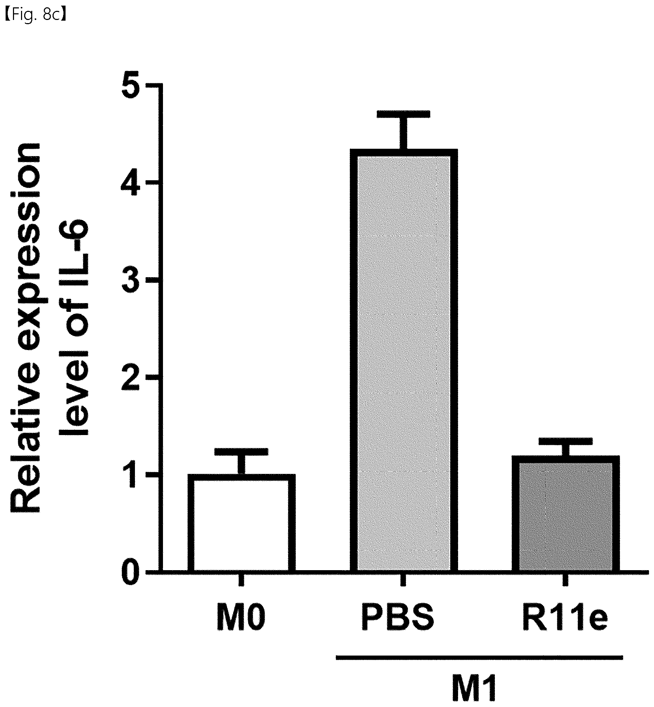

Thereafter, TNF-α, IL-1β, and IL-6 were monitored for mRNA expression levels by real-time PCR using the synthesized cDNA and the primers of Table 11, and the results are given in FIGS. 8a to 8c and Tables 15 to 17.

**TABLE 15**

| | M0 | M1 | |
|---|---|---|---|
| | | PBS | R11e |
| Expression level of TNF-α | 1.0 | 7.2 | 2.0 |

**TABLE 16**

| | M0 | M1 | |
|---|---|---|---|
| | | PBS | R11e |
| Expression level of IL-1β | 1.0 | 5.4 | 1.7 |

**TABLE 17**

| | M0 | M1 | |
|---|---|---|---|
| | | PBS | R11e |
| Expression level of IL-6 | 1.0 | 4.3 | 1.2 |

As can be seen in FIGS. 8a to 8c and Tables 15 to 17, relative expression levels of TNF-α, IL-1β, and IL-6 were measured to decrease by about 72%, about 69%, and about 72% in BxC-R11e exosome-treated inflammatory macrophages, respectively, compared to PBS-treated control. The data obtained from the above experiments indicate that the BxC-R11e exosomes according to the present disclosure decreases levels of inflammatory cytokines expressed from inflammatory macrophages.

### 9-2. Proliferative effect on regenerative macrophage

The human monocyte cell line THP-1 was seeded into a 35-mm dish containing an RPMI1640 medium supplemented with 1% antibiotics-antimycotics, 10% FBS, and PMA (200 ng/ml) and incubated at 37°C for 2 days in a 5% CO₂ incubator. After 48 hours of incubation, the cells were observed to adhere to the dish. Subsequently, the cells were induced to differentiate into regenerative macrophages. In this regard, the medium was aspirated and the cells were washed with DPBS and then incubated at 37°C for an additional 3 days in an RPMI1640 medium supplemented with recombinant human interleukin-4 (IL-4, 20 ng/ml, Peprotech, USA), recombinant human interleukin-13 (IL-13, 20 ng/ml, Prospec, Israel), 1% antibiotics-antimycotics, and 10% FBS in a 5% CO₂ incubator. After 72 hours, the medium was removed and the cells were washed DPBS and incubated at 37°C for 24 hours in an RPMI1640 medium supplemented with 1% antibiotics-antimycotics and 50 ug/ml BxC-R11e exosomes in a 5% CO₂ incubator so as to examine an effect of the exosomes on the regenerative macrophages. Then, 1 ml of TRIZol reagent (Invitrogen, USA) was added to the 35-mm dish to lyse the cells. The cell lysate was added with 200 µl of chloroform (Sigma, USA), briefly vortexed, and centrifuged at 4°C and 12,000 rpm for 15 min. The supernatant thus formed was transferred into a new tube and mixed with 500 µl of isopropanol (Merck Millipore, USA). The tube was turned upside down 50 times, left on ice for 5 min, and centrifuged at 12,000 rpm and 4°C for 10 min. The supernatant was removed, and 1 ml of 70% ethanol was added to the pellet, followed by centrifugation at 12,000 rpm and 4°C for 5 min. The ethanol was aspirated, and the RNA pellet in the tube was dried before dissolution in nuclease-free water. The concentration of the RNA sample thus obtained was measured by Nanodrop absorbances at 260 nm and 280 nm and cDNA was synthesized therefrom using AccuPower CycleScript RT PreMix(dT₂₀) (K-2044, Bioneer, Korea).

Thereafter, CD206, ARG-1, and IL-10 were monitored for mRNA expression levels by real-time PCR using the synthesized cDNA and the primers of Table 18.

**TABLE 18**

| SEQ ID NO: | Name | Sequencing List (5'-> 3') | Note |
|---|---|---|---|
| 7 | CD206_prime r1 | CACGATCCGACCCTTCCTTG | CD206 forward primer |
| 8 | CD206_prime r2 | GCTTGCAGTATGTCTCCGCT | CD206 reverse primer |
| 9 | ARG-1_primer1 | CGGAGACCACAGTTTGGCA | ARG-1 forward primer |
| 10 | ARG-1_primer2 | CCTGGCACATCGGGAATCTT | ARG-1 reverse primer |
| 11 | IL-10_primer1 | TGAAAACAAGAGCAAGGCCG | IL-10 forward primer |
| 12 | IL-10_primer2 | GCCACCCTGATGTCTCAGTT | IL-10 reverse primer |

As can be seen in FIGS. 9a to 9c and Tables 19 to 21, it was measured that relative expression levels of CD206, ARG-1, and IL-10 did not significantly decrease in BxC-R11e exosome-treated regenerative macrophages, compared to PBS-treated control.

**TABLE 19**

| | M0 | M2 | |
|---|---|---|---|
| | | PBS | R11e |
| Expression level of CD206 | 1.0 | 76 | 60 |

**TABLE 20**

| | M0 | M2 | |
|---|---|---|---|
| | | PBS | R11e |
| Expression level of ARG-1 | 1.0 | 58 | 41 |

**TABLE 21**

| | M0 | M2 | |
|---|---|---|---|
| | | PBS | R11e |
| Expression level of IL-10 | 1.0 | 9.3 | 6.5 |

The data obtained from the above experiments indicate that the BxC-R11e exosomes according to the present disclosure maintain the levels of cytokine expressed from regenerative macrophages.

### EXPERIMENTAL EXAMPLE 10: Inhibitory Effect of BxC-R11e Exosome on Fibrosis of Fibroblast

### 10-1. Measurement of expression level by real-time PCR

The mouse embryonic fibroblast cell line CF-1 (ATCC, USA) was seeded into 6-well plates containing a high-glucose DMEM medium (Gibco, USA) supplemented with 1% antibiotics-antimycotics and 10% FBS and incubated at 37°C for 1 day in a 5% CO₂ incubator. The medium was changed with a high-glucose DMEM medium (Gibco, USA) supplemented with 20 ng/ml transforming growth factor-β1 human (TGF-β1, Sigma) and 1% antibiotics-antimycotics, followed by incubation at 37°C for 2 days in a 5% CO₂ incubator to induce fibrosis.

Then, the cells were washed with DPBS and incubated at 37°C for 1 day in a medium containing 100 µg/ml BxC-e or BxC-R11e exosomes in a 5% CO₂ incubator so as to examine the inhibitory effect of the exosomes on fibrosis.

After completion of the culturing, 1 ml of TRIZol reagent (Invitrogen, USA) was added to the 35-mm dish to lyse the cells. The cell lysate was added with 200 µl of chloroform (Sigma, USA), briefly vortexed, and centrifuged at 4°C and 12,000 rpm for 15 min. The supernatant thus formed was transferred into a new tube and mixed with 500 µl of isopropanol (Merck Millipore, USA). The tube was turned upside down 50 times, left on ice for 5 min, and centrifuged at 12,000 rpm and 4°C for 10 min. The supernatant was removed, and 1 ml of 70% ethanol was added to the pellet, followed by centrifugation at 12,000 rpm and 4°C for 5 min. The ethanol was aspirated, and the RNA pellet in the tube was dried before dissolution in nuclease-free water. The concentration of the RNA sample thus obtained was measured by Nanodrop absorbances at 260 nm and 280 nm and cDNA was synthesized therefrom using AccuPower CycleScript RT PreMix(dT₂₀) (K-2044, Bioneer, Korea).

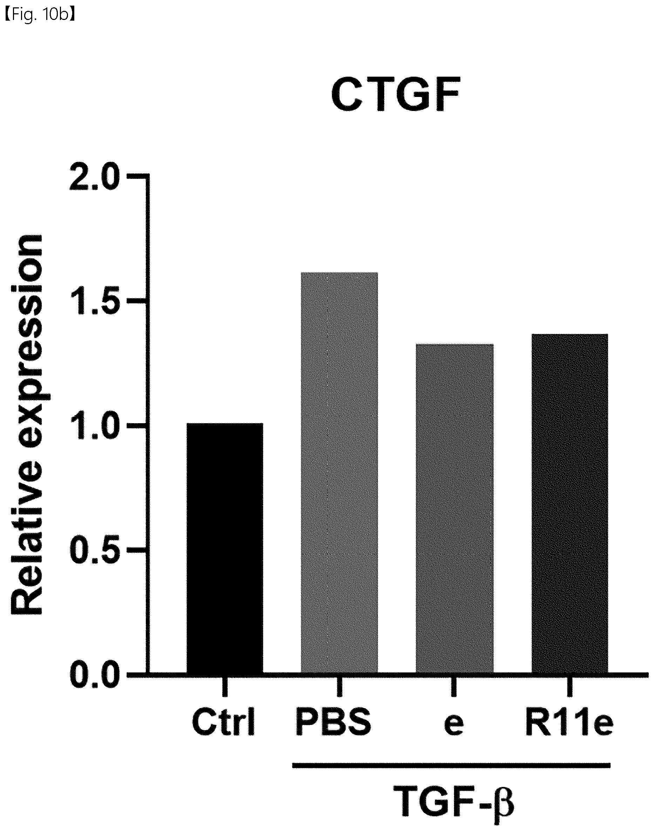

Thereafter, α-SMA (Acta2) and CTGF were monitored for mRNA expression levels by real-time PCR using the synthesized cDNA and the primers of Table 22, and the results are given in FIGS. 10a and 10b and Tables 23 to 24.

**TABLE 22**

| SEQ ID NO: | Name | Sequencing List (5'-> 3') | Note |
|---|---|---|---|
| 13 | α-SMA _primer1 | CCCAGACATCAGGGAGTAATGG | α-SMA forward primer |
| 14 | α-SMA _primer2 | TCTATCGGATACTTCAGCGTCA | α-SMA reverse primer |
| 15 | CTGF_primer1 | CTTCTGCGATTTCGGCTCC | CTGF forward |
| 16 | CTGF_primer 2 | TACACCGACCCACCGAAGA | CTGF reverse primer |

**TABLE 23**

| | Ctrl | TGF-β | | |
|---|---|---|---|---|
| | | PBS | e | R11e |
| Expression level of α-SMA | 1.0 | 1.8 | 1.0 | 1.0 |

**TABLE 24**

| | Ctrl | TGF-β | | |
|---|---|---|---|---|
| | | PBS | e | R11e |
| Expression level of CTGF | 1.0 | 1.6 | 1.3 | 1.3 |

As can be seen in FIGS. 10a and 10b and Tables 23 and 24, BxC-e and BxC-R11e exosomes were both observed to decrease the expression of the fibrosis-related genes α-SMA and CTGF in fibroblasts which had been induced to undergo fibrosis. As demonstrated by the data, the exosomes according to the present disclosure are expected to have an effect of inhibiting fibrosis of cells.

### 10-2. Measurement of expression level by SDS-PAGE

The mouse embryonic fibroblast cell line CF-1 (ATCC, USA) was seeded into 6-well plates containing a high-glucose DMEM medium (Gibco, USA) supplemented with 1% antibiotics-antimycotics and 10% FBS and incubated at 37°C for 1 day in a 5% CO₂ incubator. The medium was changed with a high-glucose DMEM medium (Gibco, USA) supplemented with 20 ng/ml transforming growth factor-β1 human (TGF-β1, Sigma) and 1% antibiotics-antimycotics, followed by incubation at 37°C for 2 days in a 5% CO₂ incubator to induce fibrosis.

Then, the cells were washed with DPBS and incubated at 37°C for 1 day in a medium containing 100 µg/ml BxC-e or BxC-R11e exosomes in a 5% CO₂ incubator so as to examine the inhibitory effect of the exosomes on fibrosis.

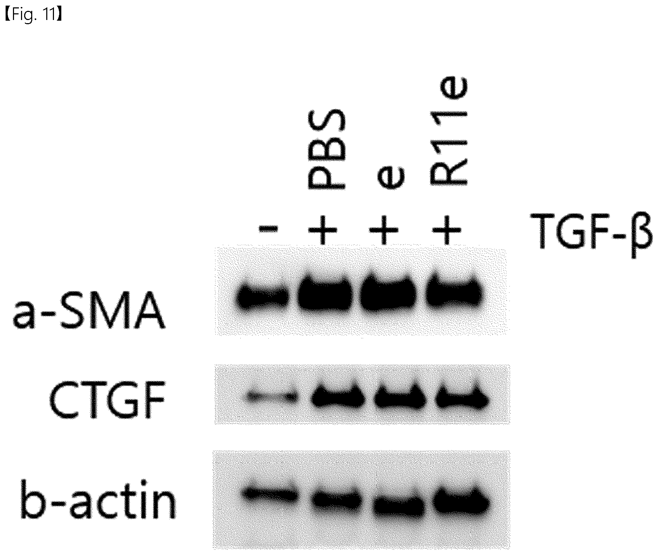

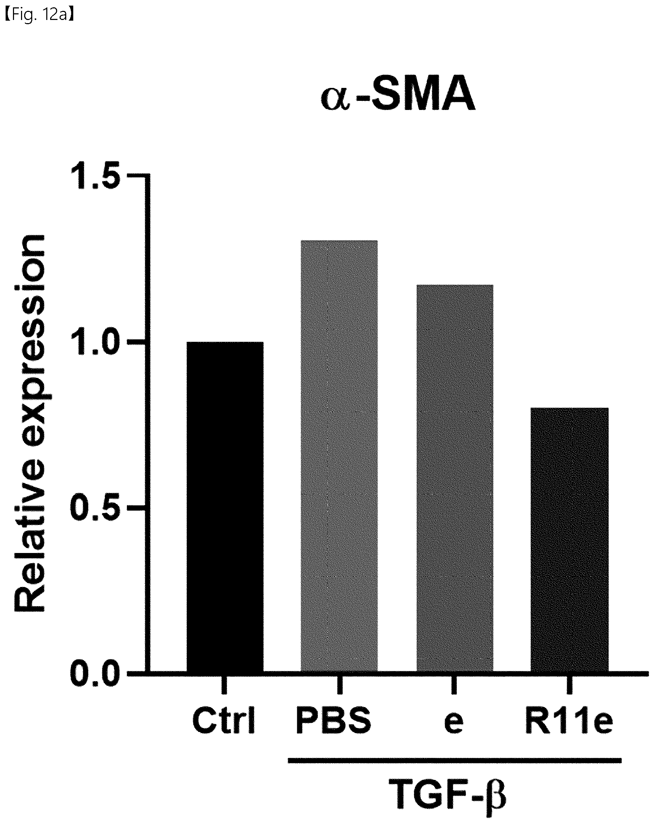

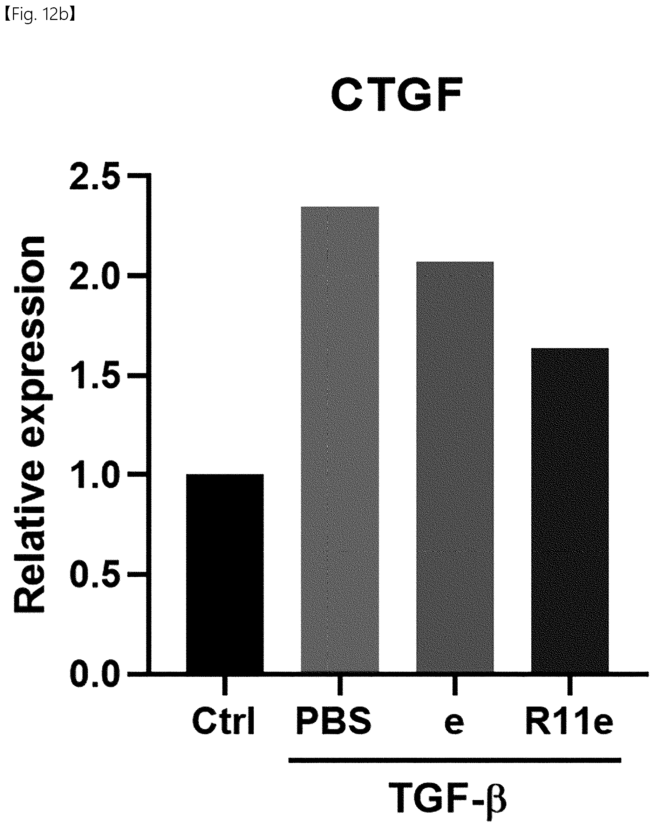

After completion of the culturing, the culture medium was aspirated and the cells were washed with DPBS, added with 0.5 ml of TryPLE(Gibco) per well in the 6-well plates, and left at 37°C for 3 min in a 5% CO₂ incubator. When the cells floated, the cell suspension was collected in a tube containing a culture medium added with FBS, and centrifuged at 5000 rpm for 3 min. The supernatant thus formed was decanted and the cell pellet was lysed with 50 µl of a NP40 cell lysis buffer (FNN0021, Invitrogen) for 30 min on ice, followed by centrifugation at 4°C and 12000 rpm for 10 min. The supernatant was collected in a new tube. After quantitation, the supernatant was diluted with a sample buffer and boiled at 100°C for 10 min. The resulting sample was subjected to SDS-PAGE and transferred onto a membrane. Antibodies to α-SMA and CTGF were added in an amount of 1/1000 of the buffer and reacted at 4°C for 16 hours. The membrane was washed with a TBST buffer and reacted at room temperature for 1 hour with a secondary antibody added in an amount of 1/2000 of the buffer. Then, the membrane was washed with a TBST buffer, followed by reaction with an ECL solution. Expression levels were measured using ChemiDoc and quantitated using the Multigauge program. The data are depicted in FIGS. 11, 12a, and 12b and summarized in Tables 25 and 26.

**TABLE 25**

| | Ctrl | TGF-β | | |
|---|---|---|---|---|
| | | PBS | e | R11e |
| Expression level of α-SMA | 1.0 | 1.3 | 1.2 | 0.8 |

**TABLE 26**

| | Ctrl | TGF-β | | |
|---|---|---|---|---|
| | | PBS | e | R11e |
| Expression level of CTGF | 1.0 | 2.3 | 2.0 | 1.6 |

Consistent with the data of the real-time PCR, as can be seen in FIGS. 11, 12a, and 12b and Tables 25 and 26, BxC-e and BxC-R11e exosomes were both observed to decrease the expression of the fibrosis-related genes α-SMA and CTGF in fibroblasts which had been induced to undergo fibrosis. As demonstrated by the data, the exosomes according to the present disclosure are expected to have an effect of inhibiting fibrosis of cells.

### EXPERIMENTAL EXAMPLE 11: Promotive Effect of BxC-e and BxC-R11e Exosomes on Cardiomyocytic Function

The iPSC-derive cardiomyocytes that had completely undergone differentiation were seeded into 6-well plates containing a RPMI1640 medium without vitamin A supplement and incubated at 37°C for 1 day in a 5% CO₂ incubator. Then, the cells were washed with DPBS and incubated at 37°C for 1 day in a medium containing 100 µg/ml BxC-e or BxC-R11e exosomes in a 5% CO₂ incubator so as to examine the promotive effect of the exosomes on cardiomyocytic functions.

After completion of the culturing, 1 ml of TRIZol reagent (Invitrogen, USA) was added to the 35-mm dish to lyse the cells. The cell lysate was added with 200 µl of chloroform (Sigma, USA), briefly vortexed, and centrifuged at 4°C and 12,000 rpm for 15 min. The supernatant thus formed was transferred into a new tube and mixed with 500 µl of isopropanol (Merck Millipore, USA). The tube was turned upside down 50 times, left on ice for 5 min, and centrifuged at 12,000 rpm and 4°C for 10 min. The supernatant was removed, and 1 ml of 70% ethanol was added to the pellet, followed by centrifugation at 12,000 rpm and 4°C for 5 min. The ethanol was aspirated, and the RNA pellet in the tube was dried before dissolution in nuclease-free water. The concentration of the RNA sample thus obtained was measured by Nanodrop absorbances at 260 nm and 280 nm and cDNA was synthesized therefrom using AccuPower CycleScript RT PreMix(dT₂₀) (K-2044, Bioneer, Korea).

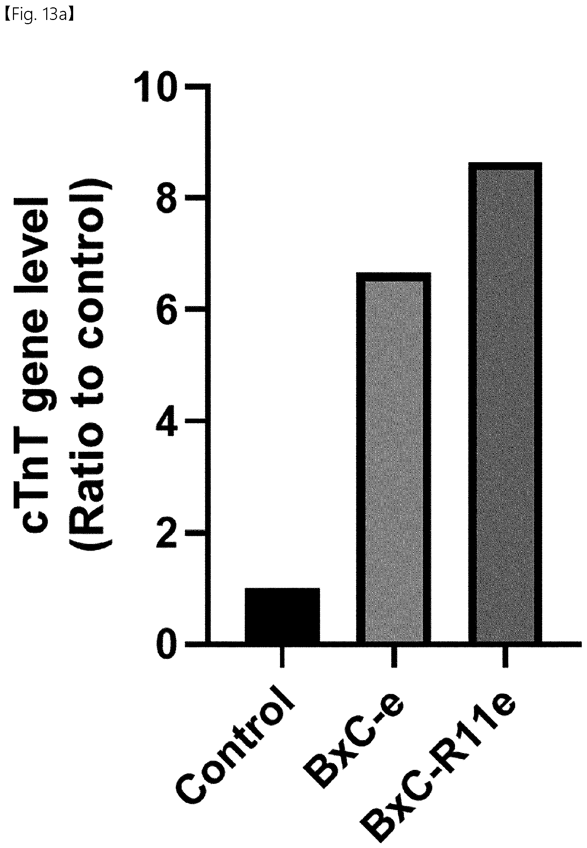

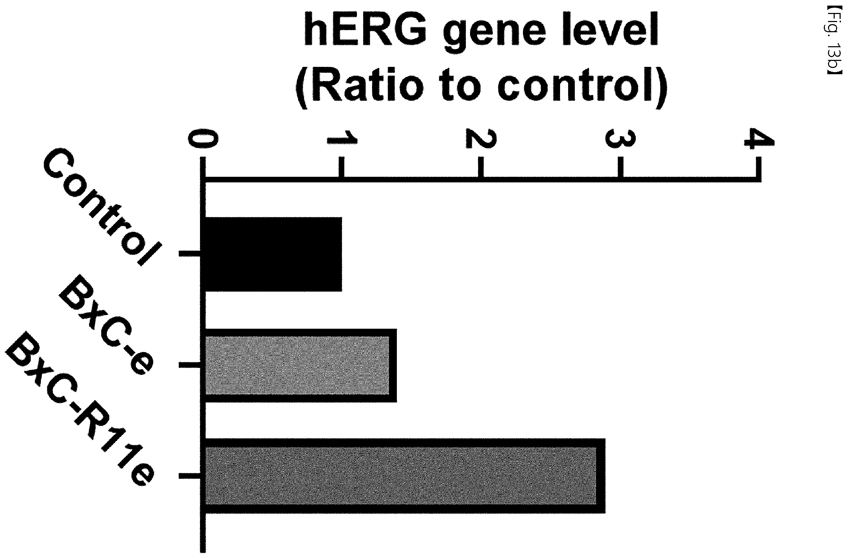

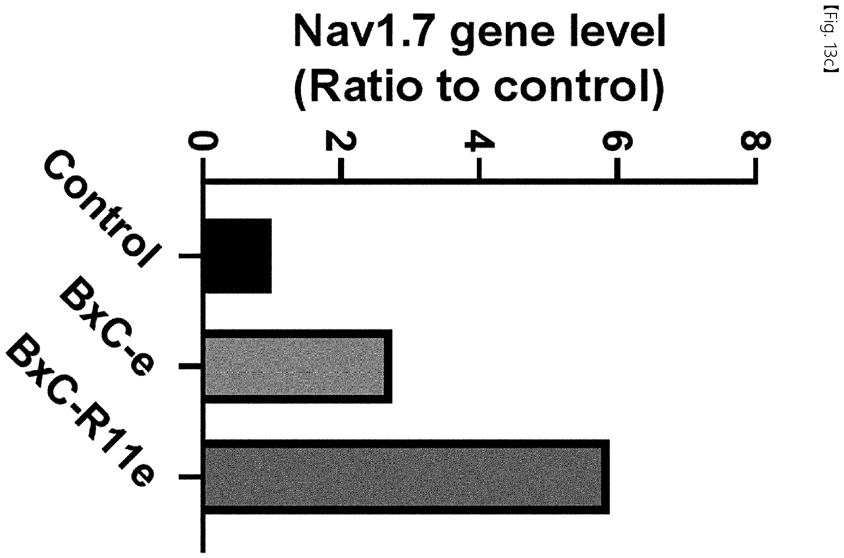

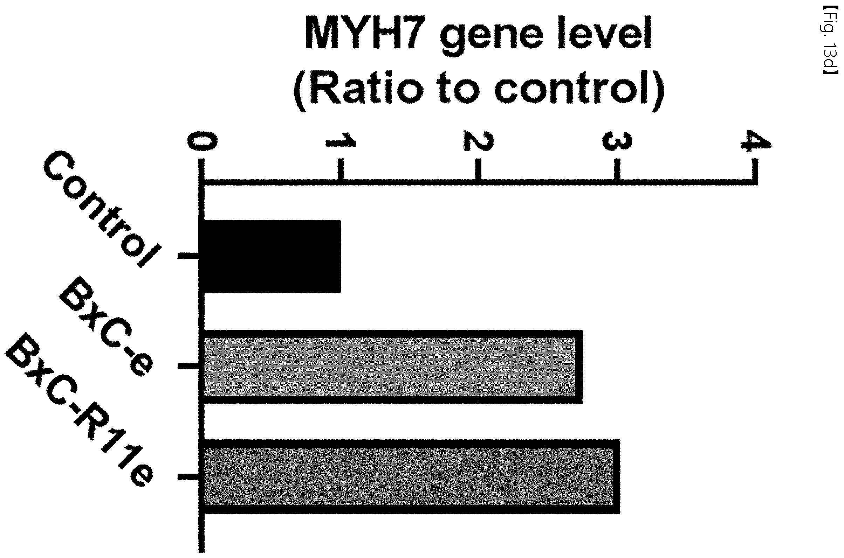

Thereafter, cTNT, hERG, Nav1.7, and MYH7 were monitored for mRNA expression levels by real-time PCR using the synthesized cDNA and the primers of Table 27, and the results are given in FIGS. 13a to 13d and Tables 28 to 31.

**TABLE 27**

| SEQ ID NO: | Name | Sequencing List (5'-> 3') | Note |
|---|---|---|---|
| 17 | cTNT_primer1 | AGTGGGAAGAGGCAGACTGA | cTNT forward primer |
| 18 | cTNT_primer2 | CGAACTTCTCTGCCTCCAAG | cTNT reverse primer |
| 19 | hERG_primer1 | CAACCTGGGCGACCAGATAG | hERG forward primer |
| 20 | hERG_primer2 | GGTGTTGGGAGAGACGTTGC | hERG reverse primer |
| 21 | Nav1.7_primer1 | GGTTTCAGCACAGATTCAGGTC | Nav1.7 forward primer |
| 22 | Nav1.7_primer2 | CCAGCTGAAAGTGTCAAAGCTC | Nav1.7 reverse primer |
| 23 | MYH7_primer1 | CTTTGCTGTTATTGCAGCCATT | MYH7 forward primer |
| 24 | MYH7_primer2 | AGATGCCAACTTTCCTGTTGC | MYH7 reverse primer |

**TABLE 28**

| | Ctrl | BxC-e | BxC-R11e |
|---|---|---|---|
| Relative expression level of cTnT | 1.0 | 6.67 | 8.65 |

**TABLE 29**

| | Ctrl | BxC-e | BxC-R11e |
|---|---|---|---|
| Relative expression level of hERG | 1.0 | 1.4 | 2.89 |

**TABLE 30**

| | Ctrl | BxC-e | BxC-R11e |
|---|---|---|---|
| Relative expression level of Nav 1.7 | 1.0 | 2.74 | 5.89 |

**TABLE 31**

| | Ctrl | BxC-e | BxC-R11e |
|---|---|---|---|
| Relative expression level of MYH7 | 1.0 | 2.76 | 3.03 |

As can be seen in FIGS. 13a to 13d and Tables 23 to 31, the cardiomyocytes treated with BxC-e and BxC-R11e exosomes were observed to greatly increase mRNA expression levels of cTNT, hERG, Nav1.7, and MYH7, compared to the control. As demonstrated by the data, BxC-e and BxC-R11e exosomes are expected to remarkably enhance the functions of cardiomyocytes when applied to cardiomyocytes that have undergone dysfunction or have been damaged.

### Industrial Applicability

The present disclosure relates to a composition including stem cell-derived exosomes and a method for producing same and, more specifically, to a composition that includes exosomes isolated from mesenchymal stem cells or a culture thereof and is superb in terms of anti-inflammatory activity, fibrosis inhibition, vascular endothelial cell proliferation, blood vessel formation, viability improvement, and protective regeneration of cardiomyocytes.

## Claims

1. A pharmaceutical composition for alleviation, suppression, prevention, or treatment of a heart disease, the composition comprising at least one selected from the group consisting of BxC stem cell-derived exosomes, BxC-A1 stem cell-derived exosomes, BxC-I10 stem cell-derived exosomes, BxC-G63 stem cell-derived exosomes, BxC-R11 stem cell-derived exosomes, and BxC-R56 stem cell-derived exosomes.

2. The pharmaceutical composition of claim 1, wherein the heart disease is selected from the group consisting of angina pectoris, myocardial infarction, valvular disease, cardiac failure, cardiac hypertrophy, arrhythmia, pericarditis, and endocarditis.

3. The pharmaceutical composition of claim 1, wherein the heart disease is an ischemic heart disease.

4. The pharmaceutical composition of claim 1, wherein the composition comprising BxC-R11 stem cell-derived exosomes.

5. A method for preparing a pharmaceutical composition for alleviation, suppression, prevention, or treatment of a heart disease, the method comprising:
a first culturing step of culturing induced pluripotent stem cells in a medium;
a selective culturing step of separating SSEA-4 (- ) cells from the cultured induced pluripotent cells and culturing same to perform differentiation into BxC stem cells;
a second culturing step of culturing the BxC stem cells to perform differentiation into mesenchymal stem cells;
a pretreatment step of pretreating the mesenchymal stem cells with at least one selected from the group consisting of pioglitazone, phorbol 12-myristate 13-acetate, exendin-4, hyaluronic acid, and resveratrol;
a production step of culturing the pretreated mesenchymal stem cells to produce exosomes; and
an isolation step of isolating exosomes from the mesenchymal stem cells or a culture thereof.

6. The method of claim 5, wherein the production step further comprises an additional culturing step of culturing the mesenchymal stem cells in presence of exosome-free fetal bovine serum (FBS).

7. A food composition for palliation, suppression, or alleviation of a heart disease, the composition comprising at least one selected from the group consisting of BxC stem cell-derived exosomes, BxC-A1 stem cell-derived exosomes, BxC-I10 stem cell-derived exosomes, BxC-G63 stem cell-derived exosomes, BxC-R11 stem cell-derived exosomes, and BxC-R56 stem cell-derived exosomes.

8. The food composition of claim 7, wherein the heart disease is selected from the group consisting of angina pectoris, myocardial infarction, valvular disease, cardiac failure, cardiac hypertrophy, arrhythmia, pericarditis, and endocarditis.

9. The food composition of claim 7, wherein the heart disease is an ischemic heart disease.

10. The food composition of claim 7, wherein the composition comprising BxC-R11 stem cell-derived exosomes.
